# EUROPEAN PATENT SPECIFICATION

(11) **EP 3 869 562 B1**
(45) Date of publication and mention of the grant of the patent: **26.11.2025**
(21) Application number: 19872819.8
(22) Date of filing: 04.10.2019
(51) Int. Cl.: H10F 39/18, G01J 1/04, H10F 77/40, A61B 1/05, A61B 1/00, H10F 39/00, G01J 1/02, G01J 3/02

(54) **SENSOR MODULE AND ELECTRONIC APPARATUS**
SENSORMODUL UND ELEKTRONISCHE VORRICHTUNG
MODULE DE CAPTEUR ET APPAREIL ÉLECTRONIQUE

(30) Priority: 15.10.2018 JP 2018194121
(43) Date of publication of application: 25.08.2021
(73) Proprietor: Sony Semiconductor Solutions Corporation, Atsugi-shi, Kanagawa 243-0014 (JP)
(72) Inventor: NAGATA, Masaya, Atsugi-shi, Kanagawa 243-0014 (JP)
(74) Representative: Müller Hoffmann & Partner
(86) International application number: PCT/JP2019/039368
(87) International publication number: WO 2020/080154

(56) References cited:
- WO-A1-2006/109638
- JP-A- 2002 006 115
- JP-A- 2004 163 960
- JP-A- 2007 158 751
- JP-A- 2007 158 751
- JP-A- 2010 045 463
- US-A1- 2004 130 640
- US-A1- 2006 076 697
- US-A1- 2010 033 647

## Description

### Technical Field

The present disclosure relates, for example, to a sensor module with an optical sensor configured as a chip-scale package and an electronic apparatus including the sensor module.

### Background Art

As sensor chips each have larger area and higher resolution, the camera modules each tend to be larger. Each of camera modules having larger area and higher resolution has an increasing peripheral distortion. This facilitates a lens module included in the camera module to have a more complicated design. An attempt is made to reduce a peripheral distortion and maintain light condensing power, for example, by adopting an aspheric lens and increasing the number of lenses. For example, PTL 1 discloses an optical device provided with a light transmissive plate on the main surface of a semiconductor element with an adhesive member interposed in between. The semiconductor element is provided with a light receiving section. The light transmissive plate has a serrated uneven section formed thereon.
US 2004/0130640 A1 relates to a solid-state imaging device that comprises a solid-state imaging element including a photo-reception portion and electrode pads, and optical glass bonded onto the solid-state imaging element through a bonding layer.
JP 2007 158751 A relates to an image pickup apparatus including a lens member with a lens and a lens support portion that supports the lens.
US 2010/0033647 A1 relates to a display element and an electronic element module in which a lens is formed as part of a translucent support substrate having a display disposed thereon.
US 2006/0076697 A1 discloses a planar lens for optical pick-ups including a transparent substrate with a lens cavity in a surface of the transparent substrate and a lens formed in the cavity.

### Citation List

### Patent Literature

PTL 1: Japanese Unexamined Patent Application Publication No. 2010-245292

### Summary of the Invention

The invention is defined by the appended claims. The following examples may include some but not all features as literally defined in the claims and are present for illustration purposes only.

Incidentally, a mobile apparatus such as a smartphone is requested to be smaller and thinner from a design perspective. For example, the camera module is requested to be reduced in height.

It is desirable to provide a sensor module and an electronic apparatus each of which allows the camera module to be reduced in height.

A sensor module according to the invention includes: a sensor substrate; and a light transmissive substrate. The sensor substrate has a light receiving surface on which a plurality of light receiving elements is arranged. The light transmissive substrate is provided on the light receiving surface. The light transmissive substrate has a flat region and an uneven region on another surface opposite to one surface opposed to the light receiving surface of the sensor substrate. The flat region has a flat front surface. The uneven region has a concave section or a convex section formed therein.

An electronic apparatus according to an example of the present disclosure includes, as a sensor module, the sensor module according to the embodiment of the present disclosure described above.

In the sensor module according to the example of the present disclosure and the electronic apparatus according to the example, the light transmissive substrate is provided on the light receiving surface of the sensor substrate. The plurality of light receiving elements is arranged on the light receiving surface. The light transmissive substrate has the flat region and the uneven region on the other surface opposite to the one surface opposed to the light receiving surface. The flat region has the flat front surface. The concave section or the convex section is formed in the uneven region. This makes it possible to use this light transmissive substrate as a lens. For example, in the camera module, the design of the lens module provided on the light receiving surface side of the sensor module is simplified. As an example, it is possible to reduce the number of lenses included in the lens module.

### Brief Description of Drawing

[FIG. 1] FIG. 1 is a cross-sectional schematic diagram illustrating an example of a configuration of a sensor module according to a first embodiment of the present disclosure.
[FIG. 2] FIG. 2 is a schematic diagram illustrating a planar configuration of the sensor module illustrated in FIG. 1.
[FIG. 3] FIG. 3 is a cross-sectional schematic diagram illustrating another example of the configuration of the sensor module according to the first embodiment of the present disclosure.
[FIG. 4] FIG. 4 is a cross-sectional schematic diagram illustrating another example of the configuration of the sensor module according to the first embodiment of the present disclosure.
[FIG. 5A] FIG. 5A is a cross-sectional schematic diagram for describing a method of manufacturing the sensor module illustrated in FIG. 1.
[FIG. 5B] FIG. 5B is a cross-sectional schematic diagram illustrating a step subsequent to FIG. 5A.
[FIG. 5C] FIG. 5C is a cross-sectional schematic diagram illustrating a step subsequent to FIG. 5B.
[FIG. 5D] FIG. 5D is a cross-sectional schematic diagram illustrating a step subsequent to FIG. 5C.
[FIG. 5E] FIG. 5E is a cross-sectional schematic diagram illustrating a step subsequent to FIG. 5D.
[FIG. 5F] FIG. 5F is a cross-sectional schematic diagram illustrating a step subsequent to FIG. 5E.
[FIG. 5G] FIG. 5G is a cross-sectional schematic diagram illustrating a step subsequent to FIG. 5F.
[FIG. 5H] FIG. 5H is a cross-sectional schematic diagram illustrating a step subsequent to FIG. 5G.
[FIG. 6A] FIG. 6A is a cross-sectional schematic diagram illustrating an example of a configuration of a sensor module according to a second embodiment of the present disclosure, comprising a feature of the invention.
[FIG. 6B] FIG. 6B is a cross-sectional schematic diagram illustrating another example of the configuration of the sensor module according to the second embodiment of the present disclosure, comprising a feature of the invention.
[FIG. 7A] FIG. 7A is a cross-sectional schematic diagram illustrating an example of a configuration of a sensor module according to a third embodiment of the present disclosure.
[FIG. 7B] FIG. 7B is a cross-sectional schematic diagram illustrating another example of the configuration of the sensor module according to the third embodiment of the present disclosure.
[FIG. 8A] FIG. 8A is a cross-sectional schematic diagram illustrating another example of the configuration of the sensor module according to the third embodiment of the present disclosure.
[FIG. 8B] FIG. 8B is a cross-sectional schematic diagram illustrating another example of the configuration of the sensor module according to the third embodiment of the present disclosure.
[FIG. 9] FIG. 9 is a cross-sectional schematic diagram illustrating an example of a configuration of a sensor module according to a fourth embodiment of the present disclosure.
[FIG. 10] FIG. 10 is a cross-sectional schematic diagram illustrating an example of a configuration of a sensor module according to a modification example 1 of the present disclosure.
[FIG. 11] FIG. 11 is a cross-sectional schematic diagram illustrating an example of a configuration of a sensor module according to a modification example 2 of the present disclosure.
[FIG. 12] FIG. 12 is a cross-sectional schematic diagram illustrating an example of a configuration of a sensor module according to a modification example 3 of the present disclosure.
[FIG. 13] FIG. 13 is a block diagram illustrating a configuration of the sensor module illustrated in FIG. 1.
[FIG. 14] FIG. 14 is a functional block diagram illustrating an example of an electronic apparatus (camera) including an imaging device illustrated in FIG. 13.
[FIG. 15] FIG. 15 is a block diagram depicting an example of a schematic configuration of an in-vivo information acquisition system.
[FIG. 16] FIG. 16 is a view depicting an example of a schematic configuration of an endoscopic surgery system.
[FIG. 17] FIG. 17 is a block diagram depicting an example of a functional configuration of a camera head and a camera control unit (CCU).
[FIG. 18] FIG. 18 is a block diagram depicting an example of schematic configuration of a vehicle control system.
[FIG. 19] FIG. 19 is a diagram of assistance in explaining an example of installation positions of an outside-vehicle information detecting section and an imaging section.

### Modes for Carrying Out the Invention

The following describes an embodiment of the present disclosure in detail with reference to the drawings. The following description is a specific example of the present disclosure, but the present disclosure is not limited to the following modes. In addition, the present disclosure is not also limited to the disposition, dimensions, dimension ratios, and the like of the respective components illustrated in the respective diagrams. It is to be noted that description is given in the following order.
1. First Embodiment (Example in which light transmissive substrate having flat region and uneven region on light incidence surface side is bonded to light receiving surface of sensor sub strate)
   1-1. Configuration of Sensor Module
   1-2. Method of Manufacturing Sensor Module
   1-3. Workings and Effects
2. Second Embodiment (Example in which flattening member is provided in uneven region on light transmissive substrate to flatten front surface)
3. Third Embodiment (Example in which functional layer is provided to light incidence surface of light transmissive substrate)
4. Fourth Embodiment (Example in which cavity is provided on light receiving surface)
5. Modification Examples
   5-1. Modification Example 1 (Example of Fan-out WCSP)
   5-2. Modification Example 2 (Another Example of Fan-out WCSP)
   5-3. Modification Example 3 (Example of sensor module having wiring structure in which no TSV is used)
6. Application Examples
7. Practical Application Examples

### <1. First Embodiment>

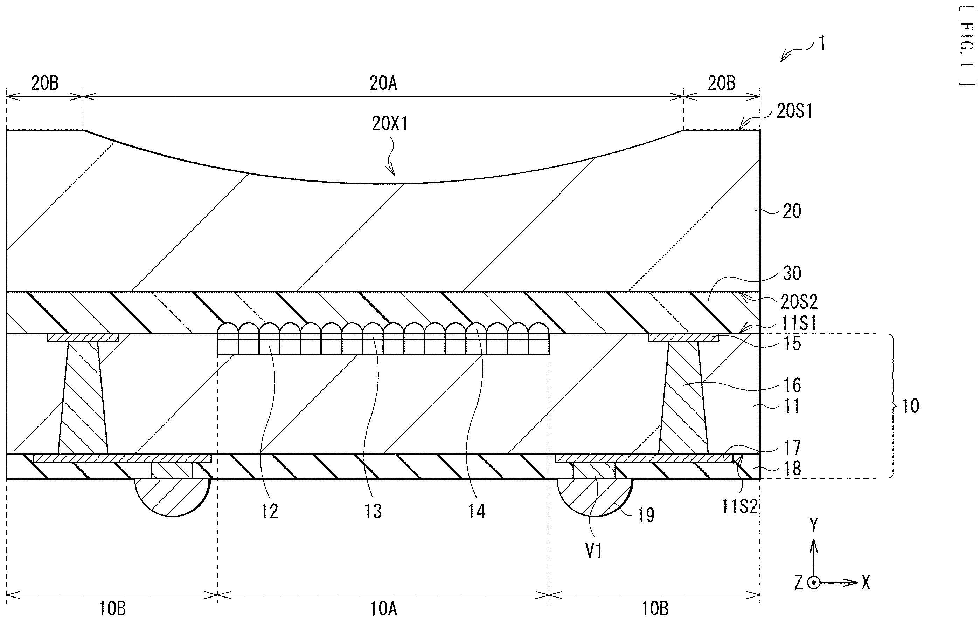

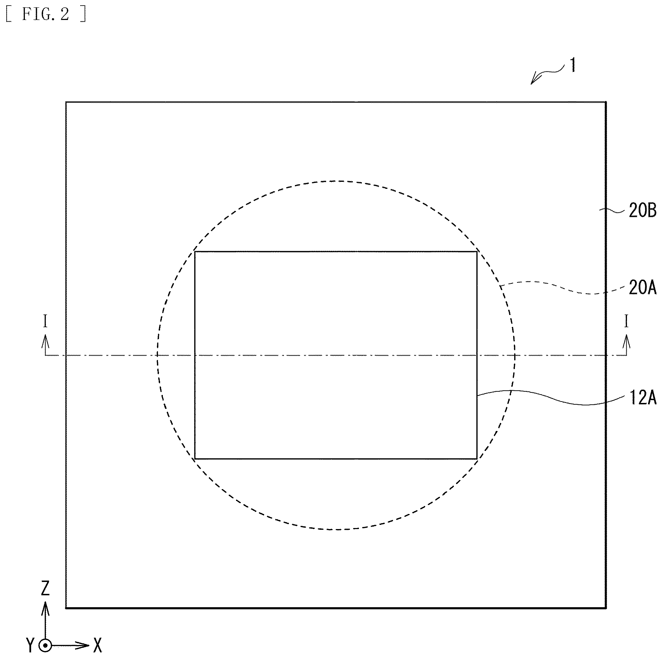

FIG. 1 schematically illustrates a cross-sectional configuration of a sensor module (sensor module 1) according to a first embodiment of the present disclosure. FIG. 2 schematically illustrates a planar configuration of the sensor module 1 illustrated in FIG. 1. FIG. 1 illustrates a portion of the cross-sectional configuration taken along the line I-I illustrated in FIG. 2. The sensor module 1 is, for example, a back-illuminated (back light receiving) CCD (Charge Coupled Device) image sensor, CMOS (Complementary Metal Oxide Semiconductor) image sensor, or the like. The sensor module 1 according to the present embodiment has a so-called wafer chip scale package (Wafer Chip Scale Package; WCSP) structure in which a light transmissive substrate 20 is bonded onto a sensor substrate 10 with a resin layer 30 interposed in between for packaging. A plurality of light receiving elements 12 is arranged on the sensor substrate 10. The light transmissive substrate 20 according to the present embodiment has a configuration in which the light transmissive substrate 20 is provided that is provided with a flat region 20B having a flat front surface and an uneven region 20A on a light incidence surface (surface 20S1; another surface) opposite to the opposed surface (surface 20S2; one surface) to the sensor substrate 10. The uneven region 20A is provided, for example, with a concave section 20X1.

### (1-1. Configuration of Sensor Module)

The sensor module 1 has a configuration in which the sensor substrate 10, the resin layer 30, and the light transmissive substrate 20 are stacked in this order.

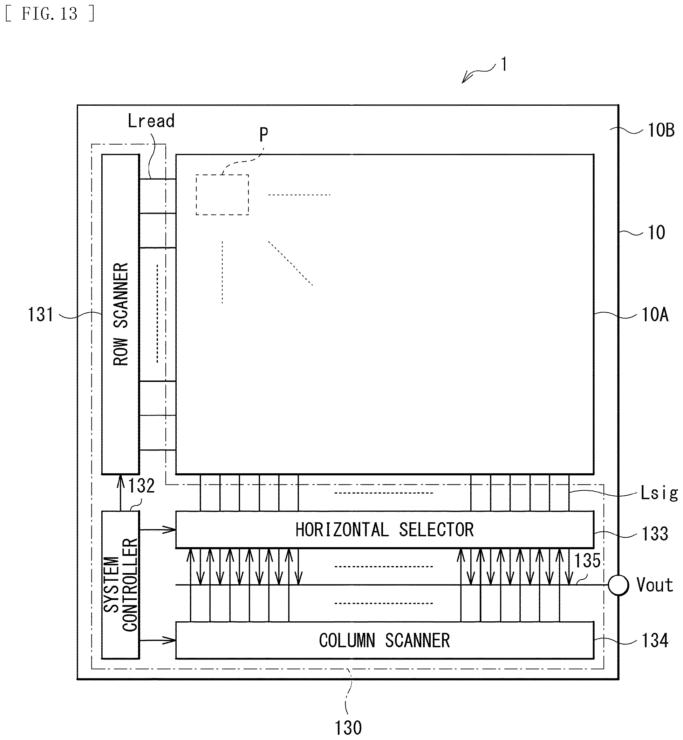

The sensor substrate 10 uses, for example, the back surface of a semiconductor substrate 11 as a light receiving surface (surface 11S1). The sensor substrate 10 has a light receiving region 10A and a peripheral region 10B. The plurality of light receiving elements 12 is arranged in an array in the light receiving region 10A. The peripheral region 10B is provided around the light receiving region 10A. In the light receiving region 10A, the light receiving elements 12 disposed at respective unit pixels P (see, for example, FIG. 13) selectively detect and photoelectrically convert respective pieces of light in different wavelength ranges. The peripheral region 10B is provided, for example, with peripheral circuits (peripheral circuit sections 130) including a row scanner 131, a horizontal selector 133, a column scanner 134, and a system controller 132 (see, for example, FIG. 13).

The semiconductor substrate 11 has the plurality of light receiving elements 12 formed, for example, in a predetermined region of an n-type silicon (Si) layer. This surface (back surface) provided with the light receiving elements 12 serves as the light receiving surface (surface 11S1). The semiconductor substrate 11 has, for example, a film thickness (simply referred to as thickness below) of 30 µm or more and 250 µm or less in a Y axis direction.

Each of the light receiving elements 12 includes, for example, a photodiode or the like. The light receiving element 12 is embedded and formed, for example, near the light receiving surface (surface 11S1) of the semiconductor substrate 11. For example, there is provided a color filter 13 on the light receiving element 12. As the color filters 13, color filters of three primary colors are arranged, for example, in the light receiving region 10A in a Bayer manner. The three primary colors include red (R), green (G), and blue (B).

There is further provided an on-chip lens 14 over the light receiving element 12 with the color filter 13 interposed in between. The on-chip lens 14 is for condensing incident light on the light receiving element 12. The on-chip lens 14 includes a material having light transmissivity. Examples of the material having light transmissivity include a transparent resin material such as an acrylic material, silicon oxide (SiO), silicon nitride (SiN), silicon oxynitride (SiON), and the like. The on-chip lens 14 includes a single-layer film including any of them or a stacked film including two or more thereof.

It is to be noted that there may be provided another layer between the light receiving element 12 and the on-chip lens 14. For example, there may be provided an anti-reflection film or an optical filter such as an infrared cut filter on the color filter 13.

There is provided a wiring line 17, for example, along with a pixel transistor on the front surface (surface (surface 11S2) opposite to the light receiving surface (surface 11S1)) side of the semiconductor substrate 11. The wiring line 17 is electrically coupled to a pad electrode 15 via a through electrode 16. The through electrode 16 penetrates, for example, the semiconductor substrate 11 between the light receiving surface (surface 11S1) and the front surface (surface 11S2). The pad electrode 15 is provided, for example, in the peripheral region 10B on the light receiving surface (surface 11S1). There is provided an insulating layer 18 on the wiring line 17. The wiring line 17 is coupled to a solder ball 19 through a via V1. The solder ball 19 is provided on the insulating layer 18. The via V1 is formed in the insulating layer 18. Further, the wiring line 17 is electrically coupled, for example, to an external circuit (not illustrated) via the solder ball 19.

The light transmissive substrate 20 functions as a lens that seals the light receiving region 10A on the sensor substrate 10 and adjusts the diffusion of incident light on the light receiving region 10A. The light transmissive substrate 20 according to the present embodiment is provided with the uneven region 20A and the flat region 20B on the light incidence surface (surface 20S1) opposite to the surface (surface 20S2) opposed to the light receiving surface (surface 11S1) of the semiconductor substrate 11. The flat region 20B has a flat front surface. The uneven region 20A is provided, for example, at the position opposed to the light receiving region 10A on the sensor substrate 10. The plurality of light receiving elements 12 is arranged in an array in the light receiving region 10A. The flat region 20B is provided around the uneven region 20A. In other words, as illustrated in FIG. 2, the uneven region 20A is provided to cover the light receiving region 10A in a plan view. Specifically, the uneven region 20A has the outer end match with the outermost end of the light receiving region 10A or has the outermost end of the light receiving region 10A disposed inside the uneven region 20A. In the present embodiment, for example, the uneven region 20A is provided with the concave section 20X1.

The light transmissive substrate 20 includes a member having light transmissivity. For example, a glass substrate is used, but this is not limitative. For example, an acrylic resin substrate, a sapphire substrate, or the like may be used for the light transmissive substrate 20. Further, it is preferable that the light transmissive substrate 20 function as a support substrate for the sensor substrate 10. In accordance with the claimed invention, the light transmissive substrate 20 has a thickness equal to or greater than that of the sensor substrate 10. It is to be noted that the thickness of the light transmissive substrate 20 serves as a distance (h) between the opposed surface (surface 20S2) to the sensor substrate 10 and the flat region 20B on the light incidence surface (surface 20S1). The upper limit of the thickness of the light transmissive substrate 20 is not particularly defined, but the light transmissive substrate 20 has ten times or less as great a thickness as that of the sensor substrate 10.

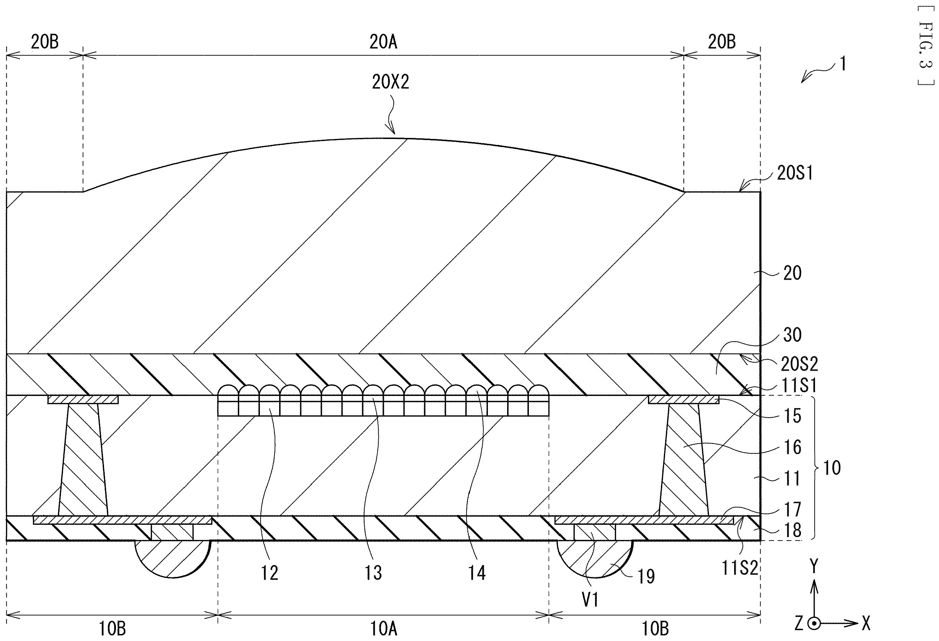

In addition, the uneven region 20A on the light transmissive substrate 20 is not limited to the concave section 20X1 as illustrated in FIG. 1. For example, a convex section 20X2 may be formed as illustrated in FIG. 3. For example, in a case where it is desirable to diffuse incident light coming to the light incidence surface (surface 20S1), it is preferable to form the concave section 20X1 illustrated in FIG. 1. In a case where it is desirable to make incident light coming to the light incidence surface (surface 20S1) converge, it is preferable to form the convex section 20X2 as illustrated in FIG. 3.

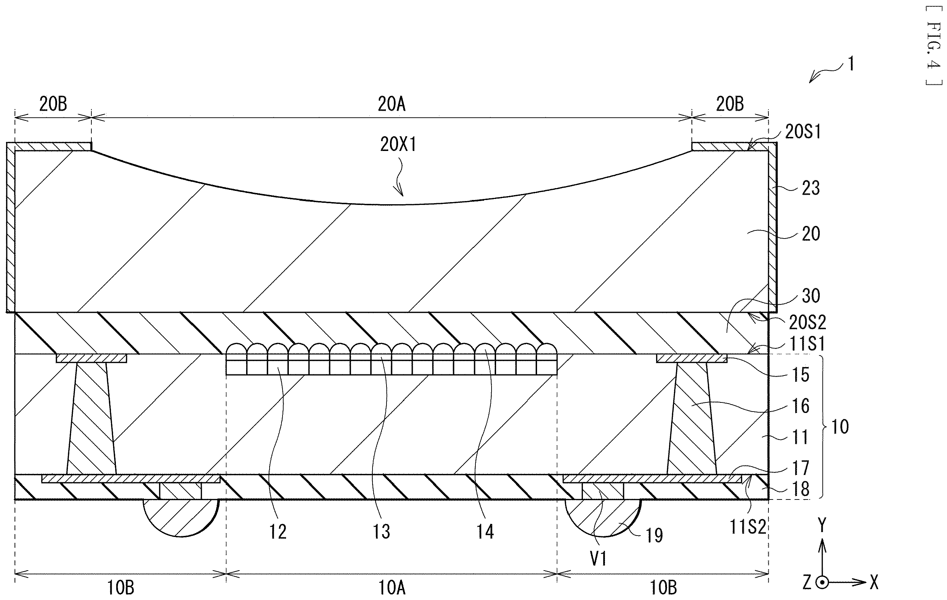

Further, the light transmissive substrate 20 may be provided, for example, with a light shielding film 23 on the side surface and the flat region 20B as illustrated in FIG. 4. It is to be noted that the light shielding film 23 does not necessarily have to be continuously provided to the flat region 20B and the side surface as illustrated in FIG. 4. The light shielding film 23 may be provided to only the flat region 20B or the side surface as appropriate. For example, it is possible to use tungsten (W), aluminum (Al), copper (Cu), titanium (Ti), nickel (Ni), and the like for the light shielding film 23. For example, it is possible to form the light shielding film 23 by sputtering, deposition, CVD, or the like.

The resin layer 30 is for bonding the sensor substrate 10 and the light transmissive substrate 20 together in a region including at least the light receiving region 10A. A resin material with optical characteristics (such as a refractive index and an extinction coefficient) prioritized is selected for the resin layer 30, for example, to allow the light receiving element 12 to favorably receive light incident on the sensor module 1. Examples of the material of the resin layer 30 include a siloxane-based resin material, an acrylic-based resin material, a styrene-based resin material, an epoxy-based resin material, and the like. Alternatively, a configuration may be adopted in which an inorganic film such as silicon oxide (SiO) or silicon nitride (SiN) is used as the resin layer 30 in place of the resin of the organic material to join the sensor substrate 10 and the light transmissive substrate 20 together.

### (1-2. Method of Manufacturing Sensor Module)

It is possible to manufacture the sensor module 1 according to the present embodiment, for example, as follows.

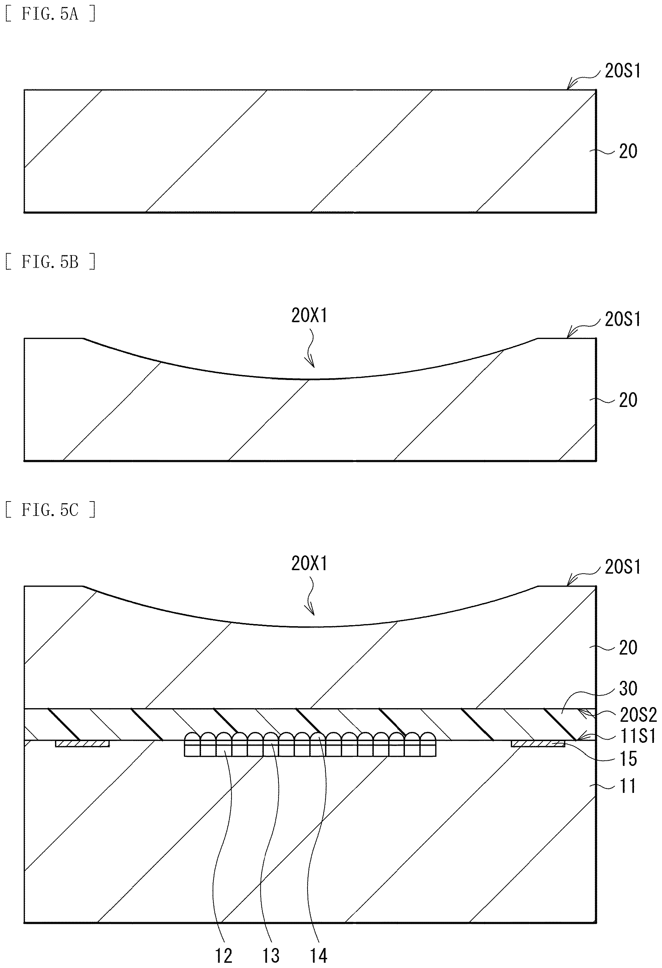

FIGs. 5A to 5H illustrate a method of manufacturing the sensor module 1 in order of steps. First, the light transmissive substrate 20 is prepared as illustrated in FIG. 5A. Next, as illustrated in FIG. 5B, for example, the concave section 20X1 having a predetermined curved surface is formed on one (surface 20S1) of the opposed surfaces by griding the glass on the surface. The curved surface has a shape corresponding to the light condensing characteristic, the size of the sensor module 1, or the configuration of the lens module (not illustrated). Examples of the grinding method include polishing, lapping, and the like. Any technique may be adopted as long as the technique makes mirror polishing possible. In addition, the abrasive grain includes diamond, alumina, carbide, nitride, garnet, cerium oxide, zirconium, and the like. In a case where glass is polished, polishing is performed to cause the light receiving region 10A to have the same diagonal line as the ground surface or cause the ground surface to be longer than the diagonal line of the light receiving region 10A to allow the light receiving region 10A to be covered.

Next, as illustrated in FIG. 5C, the light transmissive substrate 20 and the semiconductor substrate 11 included in the sensor substrate 10 are bonded together with the resin layer 30 interposed in between. It is to be noted that, in a case where the light transmissive substrate 20 and the semiconductor substrate 11 are bonded together, a protective film for protecting the front surface may be formed on the light incidence surface (surface 20S1) of the light transmissive substrate 20 or the light incidence surface (surface 20S1) of the light transmissive substrate 20 may be coated with an inorganic film. The light incidence surface (surface 20S1) is provided with the concave section 20X1.

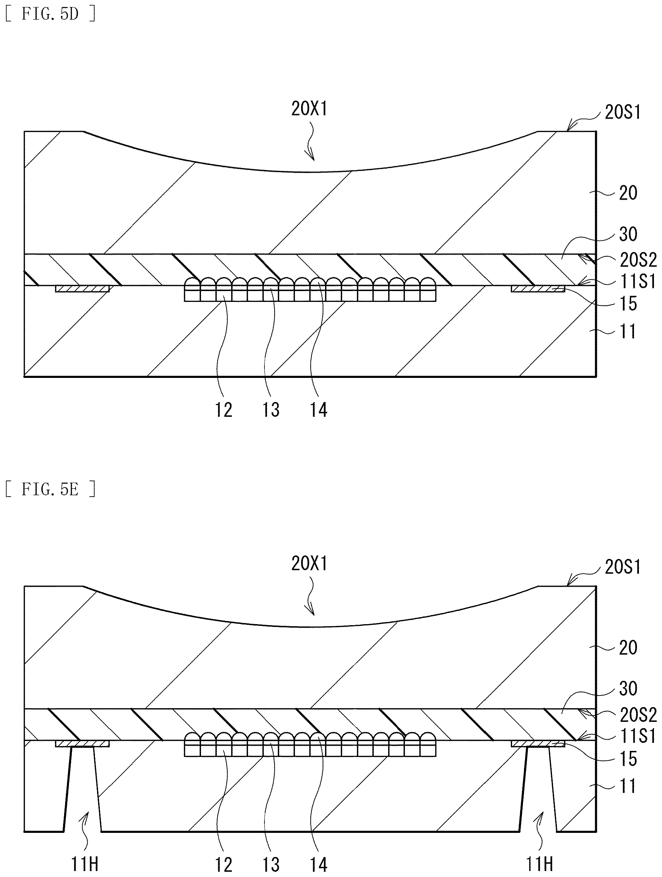

Next, as illustrated in FIG. 5D, the front surface (surface 11S2) side of the semiconductor substrate 11 is reduced in thickness, for example, by BGR (Back Grinding). For example, this thickness reduction is for facilitating the semiconductor substrate 11 to be processed in a step of forming a through electrode (TSV) performed below or increasing the coverage of the side surface and the bottom surface of an opening 11H in steps of forming an insulating layer 18A and a seed layer 17A.

Next, as illustrated in FIG. 5E, the opening 11H is formed. The opening 11H penetrates the semiconductor substrate 11 between the front surface (surface 11S1) and the light receiving surface (surface 11S1). The pad electrode 15 provided on the light receiving surface (surface 11S1) is exposed from the bottom surface of the opening 11H. It is possible to form the opening 11H, for example, by using dry etching such as reactive ion etching (RIE) or deep RIE (DRIE) or wet etching.

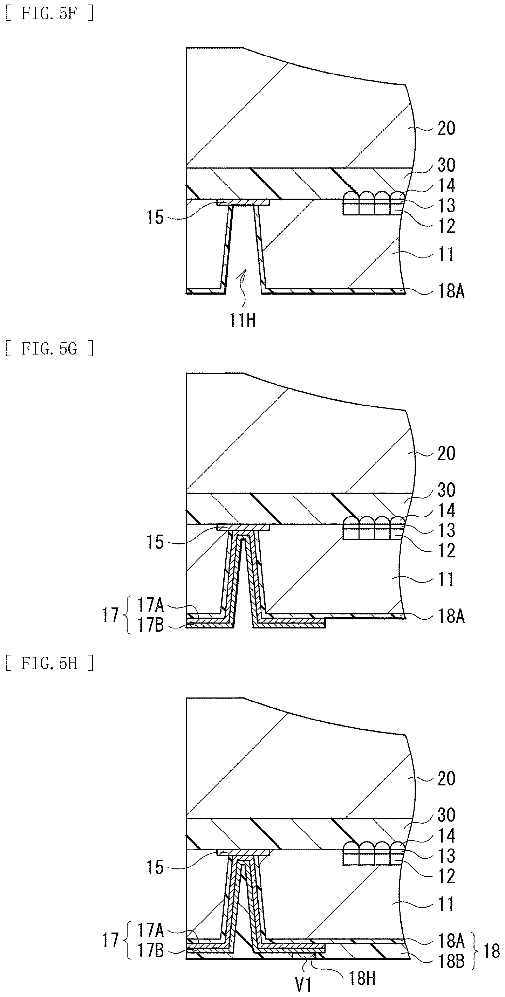

Next, as illustrated in FIG. 5F, the insulating layer 18A is formed on the back surface (surface 11S2) of the semiconductor substrate 11 and the side surface of the opening 11H. Specifically, first, the insulating layer 18A is formed on the back surface (surface 11S2) and the side surface and the bottom surface of the opening 11H. It is possible to form the insulating layer 18A, for example, by using CVD (Chemical Vapor Deposition), the lamination of an organic film, a coating method, and the like. It is to be noted that the organic film may have photosensitivity or may be non-photosensitive. Next, the insulating layer 18A formed on the bottom surface of the opening 11H is removed by full-surface etching back. The etching back is performed by using dry etching. Additionally, in a case where the insulating layer 18A includes a photosensitive organic film, the etching back may be performed by using photolithography. In a case where the insulating layer 18A includes a non-photosensitive organic film, the etching back may be performed by using a laser drill or the like.

Next, as illustrated in FIG. 5G, the wiring line 17 is formed that covers a portion of the back surface (surface 11S2) of the semiconductor substrate 11 and the side surface and the bottom surface of the opening 11H. The wiring line 17 includes, for example, the seed layer 17A and a copper (Cu) film 17B. The seed layer 17A includes a stacked film of Ti/Cu. Specifically, first, titanium (Ti) that is a barrier metal is formed on the insulating layer 18A as the seed layer 17A and then a film of copper (Cu) is further formed to form a stacked film of Ti/Cu. Ti and Cu are formed, for example, by using sputtering and deposition. Next, a resist is formed on the back surface (surface 11S2) of the semiconductor substrate 11 and patterned as the wiring line 17. Next, the Cu film 17B is formed, for example, by using electroplating and the resist is peeled off. The seed layer 17A including the stacked film of Ti/Cu and the Cu film 17B are etched. This forms the wiring line 17 that covers a portion of the back surface (surface 11S2) of the semiconductor substrate 11 and the side surface and the bottom surface of the opening 11H.

Next, as illustrated in FIG. 5H, the opening 11H is filled and a solder mask 18B is formed that covers the back surface (surface 11S2) of the semiconductor substrate 11. There is provided an opening 18H at the position corresponding to the wiring line 17 provided on the back surface (surface 11S2) of the semiconductor substrate 11. This opening 18H is plated and filled, for example, with Cu to provide the via V1. The opening 18H penetrates the solder mask 18B. Finally, the solder ball 19 is formed on the via V1 and dicing is performed for singulation. As described above, the sensor module 1 illustrated in FIG. 1 is completed.

### (1-3. Workings and Effects)

As described above, as sensor chips each have larger area and higher resolution, the camera modules each tend to be larger. Each of camera modules having larger area and higher resolution has an increasing peripheral distortion. An attempt to address an increasing peripheral distortion is made, for example, by adopting an aspheric lens or increasing the number lenses. This complicates the design of the lens module. Meanwhile, a mobile apparatus such as a smartphone is requested to be smaller and thinner from a design perspective. The camera module is requested to be reduced in height.

For this, in the sensor module 1 according to the present embodiment, the light transmissive substrate 20 is bonded to the light receiving surface (surface 11S1) of the sensor substrate 10 with the resin layer 30 interposed in between. The plurality of light receiving elements 12 is arranged on the light receiving surface (surface 11S1). The light transmissive substrate 20 has the uneven region 20A and the flat region 20B on the light incidence surface (surface 20S1). The concave section 20X1 or the convex section 20X2 is formed in the uneven region 20A. The flat region 20B has a flat front surface. This makes it possible to use this light transmissive substrate 20 as a lens. For example, it is possible in the camera module to simplify the design of the lens module provided on the light receiving surface side of the sensor module. As an example, it is possible to reduce the number of lenses included in the lens module.

As described above, in the sensor module 1 according to the present embodiment, the light transmissive substrate 20 partially has the lens module function. This reduces the number of layers of lenses included in the lens module and makes it possible to achieve a reduction in the height of the camera module.

In addition, the complicated design of the lens module described above raises problems with technical difficulty in manufacturing and resultant decreasing manufacturing yields. For this, it is possible to simplify the design of the lens module in the present embodiment. This makes it possible to increase manufacturing yields.

Further, it is possible in the sensor module 1 according to the present embodiment to sufficiently elicit sensor performance, for example, by forming the concave section 20X1 or the convex section 20X2 in the uneven region 20A on the light transmissive substrate 20. For example, in a case where the uneven region 20A is provided with the convex section 20X2, it is possible to expand the optical axis of incident light. This makes it possible to apply a small lens module to a sensor having large area.

Still further, in the present embodiment, the flat region 20B having a flat front surface is provided around the uneven region 20A. This makes it possible to increase the usability of the light transmissive substrate 20 and the sensor module 1.

Next, second to fourth embodiments and modification examples (modification examples 1 to 3) are described. It is to be noted that the components corresponding to those of the sensor module 1 according to the first embodiment are denoted by the same signs and the descriptions thereof are omitted.

### <2. Second Embodiment>

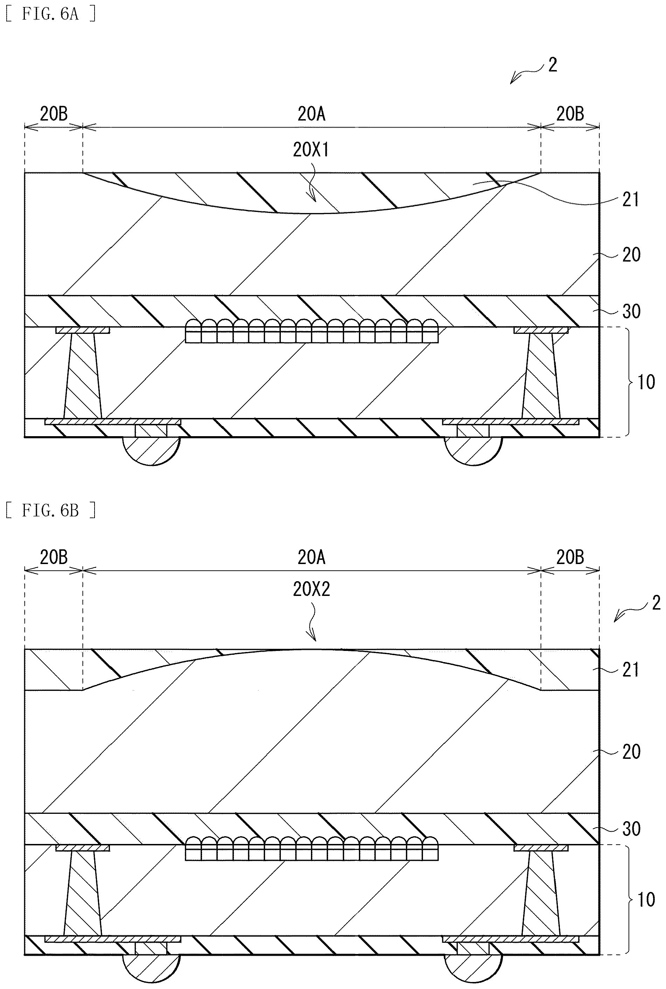

FIG. 6A schematically illustrates an example of a cross-sectional configuration of a sensor module (sensor module 2) according to a second embodiment of the present disclosure. FIG. 6B schematically illustrates another example of the cross-sectional configuration of the sensor module 2 according to the second embodiment of the present disclosure. The sensor module 2 is, for example, a back-illuminated (back light receiving) CCD image sensor, CMOS image sensor, or the like as with the sensor module 1 according to the first embodiment described above. The sensor module 2 has a so-called WCSP structure. The sensor module 2 according to the present embodiment is different from that of the first embodiment described above in that, in accordance with the claimed invention, there is provided a flattening member 21 on the light incidence surface (surface 20S1) of the light transmissive substrate 20 and the light incidence surface (surface 20S1) is flattened. The uneven region 20A and the flat region 20B that is flat are provided on the light incidence surface (surface 20S1).

The flattening member 21 is, in accordance with the invention, for filling the uneven shape formed on the light incidence surface (surface 20S1) of the light transmissive substrate 20 and flattening the front surface. The flattening member 21 is formed by using a material having light transmissivity. The material having light transmissivity has a refractive index different from that of the light transmissive substrate 20. For example, it is preferable that the flattening member 21 be formed by using a material having a lower refractive index than that of the light transmissive substrate 20. For example, in a case where the light transmissive substrate 20 includes a glass substrate having a refractive index of 1.2 to 1.9, it is preferable to use a material having a refractive index of about 1.0 for the flattening member 21. The refractive index of about 1.0 is close, for example, to the refractive index of air. Examples of such a material include a resin material such as a special acrylate, a fluororesin, or an epoxy.

It is possible to form the sensor module 2 by using a method similar to that of the first embodiment described above, for example, after forming the concave section 20X1 or the convex section 20X2 on the surface (surface 20S1) of the light transmissive substrate 20, coating the front surface with an epoxy resin, and then flattening the front surface with a squeegee or the like to form the flattening member 21. It is to be noted that the flattening member 21 may be formed, for example, by dropping an epoxy resin, for example, on the surface (surface 20S1) of the light transmissive substrate 20 at the depth of the concave section 20X1 or more, drying the epoxy resin, and then griding the resin layer to the same plane as the surface (surface 20S1) of the light transmissive substrate 20. The concave section 20X1 is formed on the surface (surface 20S1).

As described above, in the sensor module 2 according to the present embodiment, the flattening member 21 is formed on the light incidence surface (surface 20S1) of the light transmissive substrate 20 and the front surface of the light transmissive substrate 20 is flattened. This attains an effect of making it possible to handle the light transmissive substrate 20 more easily, for example, in the manufacturing step in addition to the effect of the first embodiment described above. This makes it possible to further increase, for example, the manufacturing yields.

### <3. Third Embodiment>

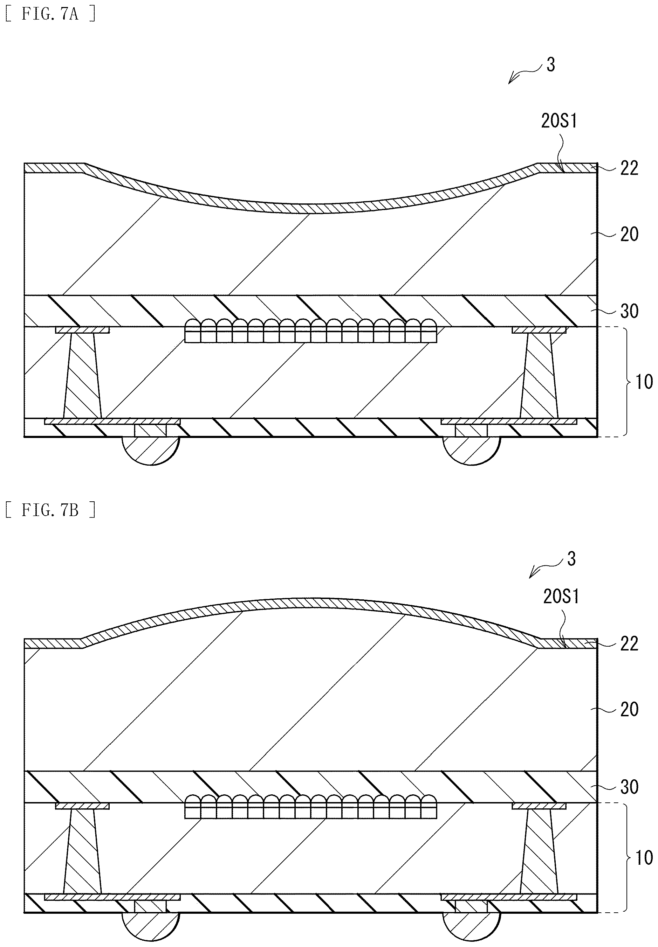

FIG. 7A schematically illustrates an example of a cross-sectional configuration of a sensor module (sensor module 3) according to a third embodiment of the present disclosure. FIG. 7B schematically illustrates another example of the cross-sectional configuration of the sensor module 3 according to the third embodiment of the present disclosure. The sensor module 3 is, for example, a back-illuminated (back light receiving) CCD image sensor, CMOS image sensor, or the like as with the sensor modules 1 and 2 according to the first embodiment and the like described above. The sensor module 3 has a so-called WCSP structure. The sensor module 3 according to the present embodiment is different from that of the first embodiment described above in that there is provided a functional layer 22 on the light incidence surface (surface 20S1) of the light transmissive substrate 20. The light incidence surface (surface 20S1) has the uneven region 20A and the flat region 20B that is flat.

The functional layer 22 is, for example, an optical film such as an anti-reflection film or an infrared cut filter, a bandpass filter, or the like. It is possible to form the anti-reflection film, for example, as a stacked film of SiO₂/TiO₂, SiO₂/Ta₂O₅, SiO₂/Al₂O₃, SiO₂/MgF₂, and the like. It is possible to form the infrared cut filter, for example, as a stacked film of SiO₂/TiO₂ or an absorbent resin film containing a dye.

As described above, in the sensor module 3 according to the present embodiment, the functional layer 22 is provided on the light incidence surface (surface 20S1) of the light transmissive substrate 20. This attains an effect of making it possible to increase the light condensing efficiency in addition to the effect of the first embodiment described above, for example, in a case where there is provided an anti-reflection film as the functional layer 22. In addition, in a case where there is provided an infrared cut filter as the functional layer 22, an effect is attained of reducing a color distortion.

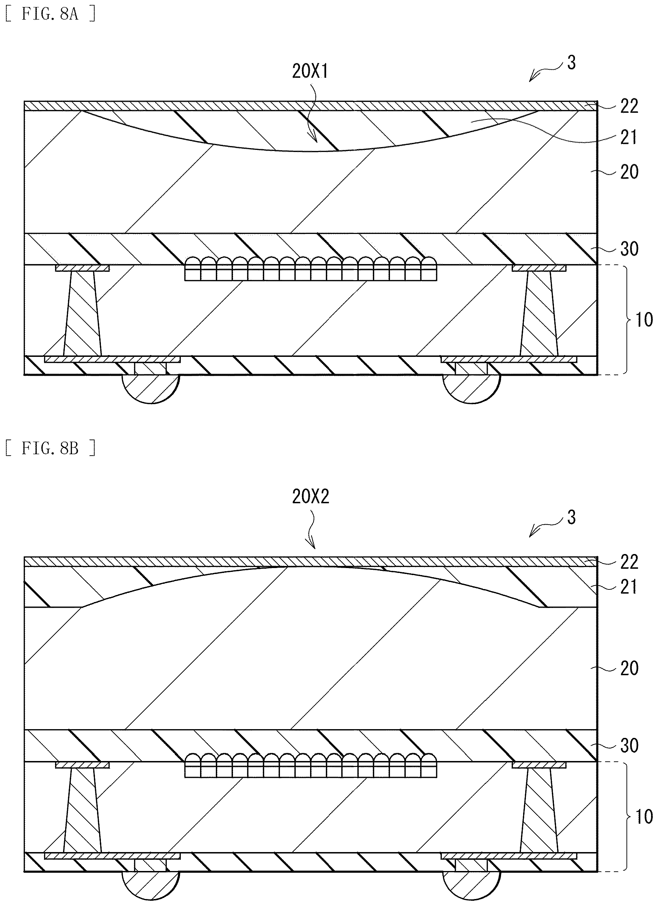

It is to be noted that the sensor module 3 according to the present embodiment may have, for example, a configuration combined with that of the second embodiment described above. For example, as illustrated in FIG. 8A, the functional layer 22 may be formed on the light incidence surface that is flattened by filling a concave section 21X1 with the flattening member 21. Alternatively, as illustrated in FIG. 8B, the functional layer 22 may be formed on the light incidence surface that is flattened by filling the flat region 20B around the convex section 20X2 with the flattening member 21.

### <4. Fourth Embodiment>

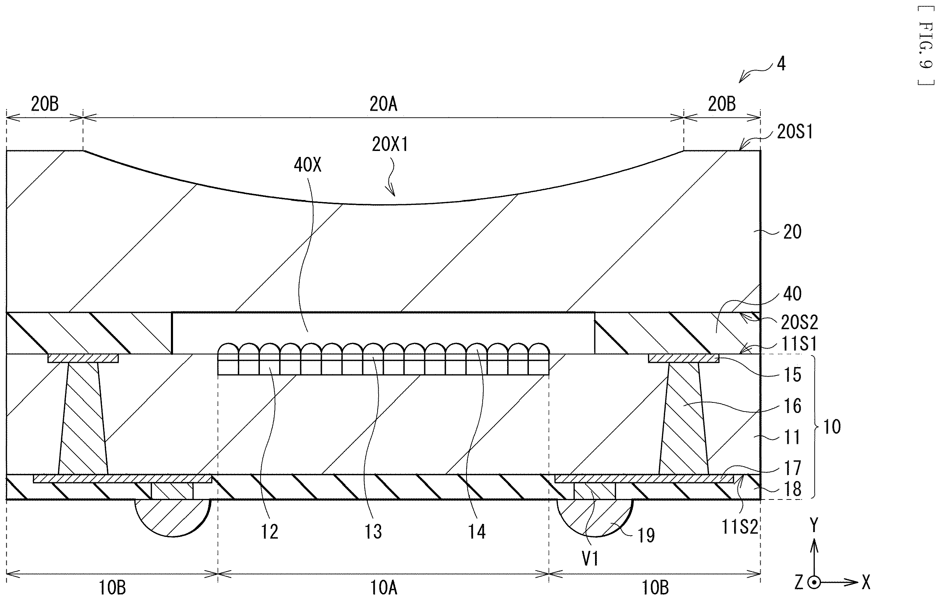

FIG. 9 schematically illustrates an example of a cross-sectional configuration of a sensor module (sensor module 4) according to a fourth embodiment of the present disclosure. The sensor module 4 is, for example, a back-illuminated (back light receiving) CCD image sensor, CMOS image sensor, or the like as with the sensor modules 1 to 3 according to the first embodiment and the like described above. The sensor module 4 has a so-called WCSP structure. The sensor module 4 according to the present embodiment is different from that of the first embodiment described above in that there is provided a resin layer 40 having a hollow structure 40X in the region corresponding to the light receiving region 10A between the sensor substrate 10 and the light transmissive substrate 20. It is to be noted that this hollow structure 40X forms a so-called cavity structure in the sensor module 4.

In this way, in the sensor module 4 having a cavity structure, the light transmissive substrate 20 is also disposed on the light receiving surface (surface 11S1) of the sensor substrate 10. This makes it possible to obtain an effect similar to that of the first embodiment described above. The light transmissive substrate 20 has the uneven region 20A and the flat region 20B on the light incidence surface (surface 20S1). The concave section 20X1 or the convex section 20X2 is formed in the uneven region 20A. The flat region 20B has a flat front surface.

### <5. Modification Examples>

### (5-1. Modification Example 1)

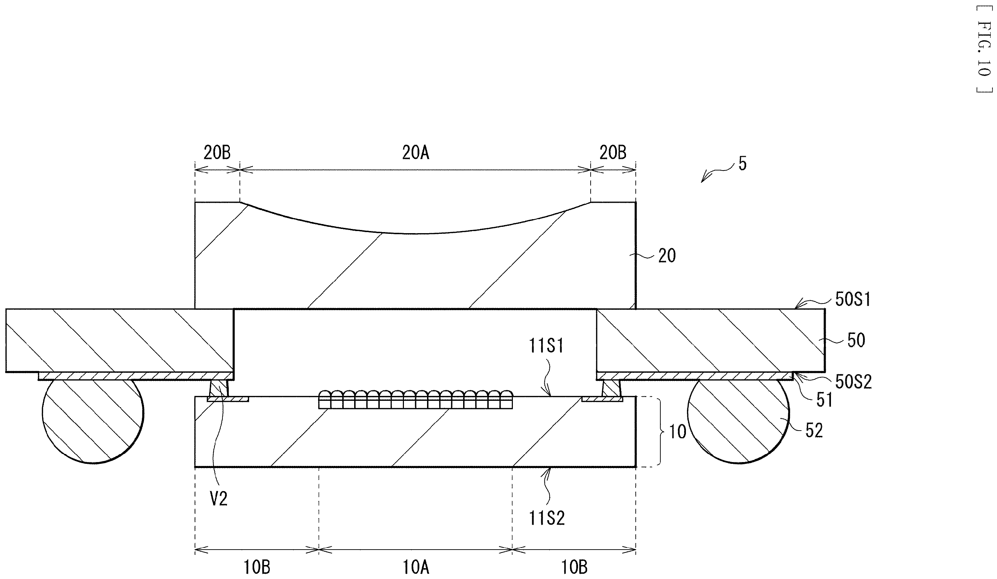

FIG. 10 schematically illustrates an example of a cross-sectional configuration of a sensor module (sensor module 5) according to a modification example (modification example 1) of the present disclosure. The sensor module 5 is, for example, a back-illuminated (back light receiving) CCD image sensor, CMOS image sensor, or the like as with the sensor modules 1 to 4 according to the first embodiment and the like described above. The sensor module 5 has a so-called Fan-out structure. The sensor module 5 according to the present modification example has a configuration in which the wiring line (wiring line 17) provided on the front surface (surface 11S2) side of the semiconductor substrate 11, for example, in the first embodiment or the like described above is separately provided on a wiring substrate 50 and this wiring substrate 50 is disposed between the sensor substrate 10 and the light transmissive substrate 20 to partially extend outside the sensor substrate 10 and the light transmissive substrate 20.

The wiring substrate 50 is provided, for example, with a wiring line 51 on the opposed surface (surface 50S2) side to the sensor substrate 10. This wiring line 51 is electrically coupled to the pad electrode 15 provided on the light receiving surface (surface 11S1) of the sensor substrate 10 through a via V2, for example, at the position opposed to the sensor substrate 10. The wiring line 51 is electrically coupled to an external circuit (not illustrated), for example, via a solder ball 52 outside the sensor substrate 10 and the light transmissive substrate 20.

### (5-2. Modification Example 2)

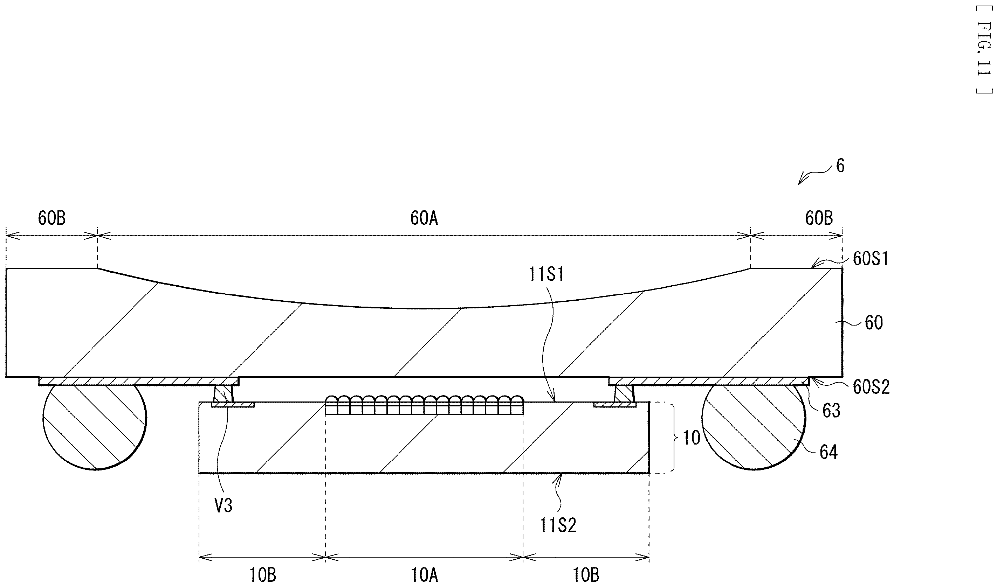

FIG. 11 schematically illustrates an example of a cross-sectional configuration of a sensor module (sensor module 6) according to a modification example (modification example 2) of the present disclosure. The sensor module 6 has a so-called Fan-out WCSP structure as in the modification example 1 described above. For example, there is provided an enlarged light transmissive substrate 60. The wiring line (wiring line 17) provided on the front surface (surface 11S2) side of the semiconductor substrate 11 in the first embodiment or the like described above is provided on a surface 60S2 of the light transmissive substrate 60.

A wiring line 63 provided on the surface 60S2 of the light transmissive substrate 60 is provided to extend from the position opposed to the peripheral region 10B on the sensor substrate 10 to the outside of the sensor substrate 10. This wiring line 63 is electrically coupled to the pad electrode 15 provided on the light receiving surface (surface 11S1) of the sensor substrate 10 through a via V3, for example, at the position opposed to the peripheral region 10B on the sensor substrate 10. The wiring line 63 is electrically coupled to an external circuit (not illustrated), for example, via a solder ball 64 outside the sensor substrate 10.

As described above, in each of the sensor modules 5 and 6 having a so-called Fan-out WCSP structure, the light transmissive substrate 20 (or light transmissive substrate 60) is also disposed on the light receiving surface (surface 11S1) of the sensor substrate 10. This makes it possible to obtain an effect similar to that of the first embodiment described above. The light transmissive substrate 20 (or light transmissive substrate 60) has the uneven region 20A (or uneven region 60A) and the flat region 20B (or flat region 60B) on the light incidence surface (surface 20S1 or surface 60S1). The flat region 20B (or flat region 60B) has a flat front surface.

### (5-3. Modification Example 3)

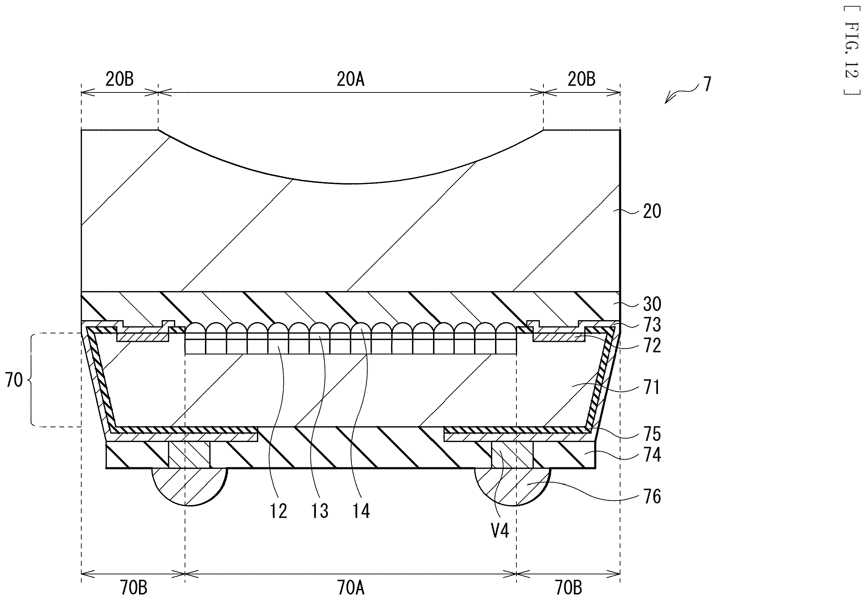

FIG. 12 schematically illustrates an example of a cross-sectional configuration of a sensor module (sensor module 7) according to a modification example (modification example 3) of the present disclosure. In the sensor module 7, a wiring line 73 is horizontally pulled out from the light receiving surface (surface 71S1) side to the front surface (surface 71S2) without using the through electrode 16.

A semiconductor substrate 71 included in a sensor substrate 70 has, for example, a trapezoidal shape. There is provided a pad electrode 72 in a peripheral region 70B on the light receiving surface (surface 71S1) of the sensor substrate 70. The wiring line 73 is coupled to the pad electrode 72. The wiring line 73 is formed on the front surface (surface 71S2) from above the pad electrode 72 via the side surface of the semiconductor substrate 71. There is provided an insulating layer 74 on the wiring line 73 on the front surface (surface 71S2) of the semiconductor substrate 71. A via V4 is embedded in the insulating layer 74 above the wiring line 73. The wiring line 73 is electrically coupled to a solder ball 76 through this via V4. The solder ball 76 is electrically coupled to an external circuit. It is to be noted that there is provided an insulating film 75 between the semiconductor substrate 71 and the wiring line 73.

In this way, in the sensor module 7 having a horizontally-pulled-out (side contact) CSP structure, the light transmissive substrate 20 is also disposed on the light receiving surface (surface 71S1) of the sensor substrate 70. This makes it possible to obtain an effect similar to that of the first embodiment described above. The light transmissive substrate 20 has the uneven region 20A and the flat region 20B on the light incidence surface (surface 20S1). The concave section 20X1 or the convex section 20X2 is formed in the uneven region 20A. The flat region 20B has a flat front surface.

### <6. Application Examples>

### (Application Example 1)

FIG. 13 is a block diagram illustrating, for example, an overall configuration of the sensor module 1 described in the first embodiment described above. This sensor module 1 is a CMOS image sensor. The sensor module 1 includes a pixel section (light receiving region 10A) as an imaging area on the sensor substrate 10. The sensor module 1 includes, for example, the peripheral circuit sections 130 in the peripheral region 10B of the light receiving region 10A. The peripheral circuit sections 130 include the row scanner 131, the horizontal selector 133, the column scanner 134, and the system controller 132.

The light receiving region 10A includes, for example, the plurality of unit pixels P (corresponding to the sensor module 1) two-dimensionally disposed in a matrix. These unit pixels P are provided with pixel drive lines Lread (specifically, row selection line and reset control line) in each of pixel rows, for example, and provided with vertical signal lines Lsig in each of pixel columns. The pixel drive lines Lread are each for transmitting drive signals for reading out signals from pixels. One end of each of the pixel drive lines Lread is coupled to the output end of the row scanner 131 corresponding to each row.

The row scanner 131 includes a shift register, an address decoder, and the like. The row scanner 131 is a pixel driving section that drives the respective unit pixels P in the light receiving region 10A, for example, row by row. Signals outputted from the respective unit pixels P in the pixel rows selectively scanned by the row scanner 131 are supplied to the horizontal selector 133 through the respective vertical signal lines Lsig. The horizontal selector 133 includes an amplifier, a horizontal selection switch, and the like provided for each of the vertical signal lines Lsig.

The column scanner 134 includes a shift register, an address decoder, and the like. The column scanner 134 drives each of the horizontal selection switches of the horizontal selector 133 in order while scanning the horizontal selection switches. As a result of this selective scanning by the column scanner 134, signals of the respective pixels to be transmitted through the respective vertical signal lines Lsig are outputted to horizontal signal lines 135 in order and are transmitted to the outside of the sensor substrate 10 through the horizontal signal lines 135.

The circuit portions including the row scanner 131, the horizontal selector 133, the column scanner 134, and the horizontal signal lines 135 may be formed directly on the sensor substrate 10 or may be provided to external control IC. In addition, those circuit portions may be formed on another substrate coupled by a cable or the like.

The system controller 132 receives a clock, data for an instruction about an operation mode, and the like that are supplied from the outside of the sensor substrate 10. In addition, the system controller 132 outputs data such as internal information of the sensor module 1. The system controller 132 further includes a timing generator that generates a variety of timing signals and controls the driving of the peripheral circuits such as the row scanner 131, the horizontal selector 133, and the column scanner 134 on the basis of the variety of timing signals generated by the timing generator.

### (Application Example 2)

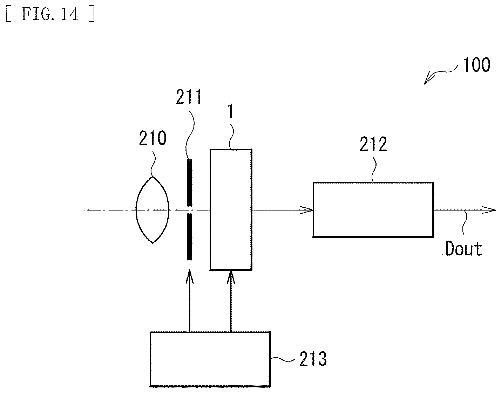

The sensor module 1 described above is applicable, for example, to any type of electronic apparatus having an imaging function including a camera system such as a digital still camera and a video camera, a mobile phone having an imaging function, and the like. FIG. 14 illustrates a schematic configuration of an electronic apparatus 100 (camera) as an example thereof. This electronic apparatus 100 is, for example, a video camera that makes it possible to shoot a still image or a moving image. The electronic apparatus 100 includes the sensor module 1, an optical system (optical lens) 210, a shutter device 211, a driver 213, and a signal processor 212. The driver 213 drives the sensor module 1 and the shutter device 211.

The optical system 210 guides image light (incident light) from an object toward a pixel section 1a of the sensor module 1. This optical system 210 may include a plurality of optical lenses. The shutter device 211 controls a period in which the sensor module 1 is irradiated with the light and a period in which the light is blocked. The driver 213 controls a transfer operation of the sensor module 1 and a shutter operation of the shutter device 211. The signal processor 212 performs various kinds of signal processing on signals outputted from the sensor module 1. An image signal Dout subjected to the signal processing is stored in a storage medium such as a memory or outputted to a monitor or the like.

Further, the sensor module 1 described above is also applicable to electronic apparatuses (a capsule type endoscope 10100 and a mobile body such as a vehicle) described below.

### <7. Practical Application Examples>

### <Practical Application Example to In-vivo Information Acquisition System>

Further, the technology (the present technology) according to the present disclosure is applicable to a variety of products. For example, the technology according to the present disclosure may be applied to an endoscopic surgery system.

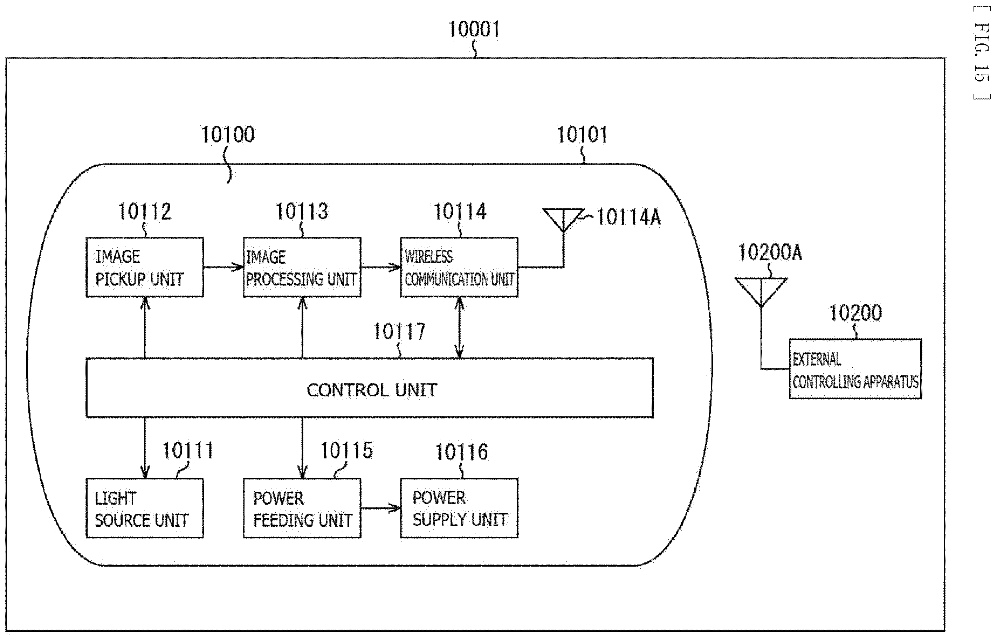

FIG. 15 is a block diagram depicting an example of a schematic configuration of an in-vivo information acquisition system of a patient using a capsule type endoscope, to which the technology according to an embodiment of the present disclosure (present technology) can be applied.

The in-vivo information acquisition system 10001 includes a capsule type endoscope 10100 and an external controlling apparatus 10200.

The capsule type endoscope 10100 is swallowed by a patient at the time of inspection. The capsule type endoscope 10100 has an image pickup function and a wireless communication function and successively picks up an image of the inside of an organ such as the stomach or an intestine (hereinafter referred to as in-vivo image) at predetermined intervals while it moves inside of the organ by peristaltic motion for a period of time until it is naturally discharged from the patient. Then, the capsule type endoscope 10100 successively transmits information of the in-vivo image to the external controlling apparatus 10200 outside the body by wireless transmission.

The external controlling apparatus 10200 integrally controls operation of the in-vivo information acquisition system 10001. Further, the external controlling apparatus 10200 receives information of an in-vivo image transmitted thereto from the capsule type endoscope 10100 and generates image data for displaying the in-vivo image on a display apparatus (not depicted) on the basis of the received information of the in-vivo image.

In the in-vivo information acquisition system 10001, an in-vivo image imaged a state of the inside of the body of a patient can be acquired at any time in this manner for a period of time until the capsule type endoscope 10100 is discharged after it is swallowed.

A configuration and functions of the capsule type endoscope 10100 and the external controlling apparatus 10200 are described in more detail below.

The capsule type endoscope 10100 includes a housing 10101 of the capsule type, in which a light source unit 10111, an image pickup unit 10112, an image processing unit 10113, a wireless communication unit 10114, a power feeding unit 10115, a power supply unit 10116 and a control unit 10117 are accommodated.

The light source unit 10111 includes a light source such as, for example, a light emitting diode (LED) and irradiates light on an image pickup field-of-view of the image pickup unit 10112.

The image pickup unit 10112 includes an image pickup element and an optical system including a plurality of lenses provided at a preceding stage to the image pickup element. Reflected light (hereinafter referred to as observation light) of light irradiated on a body tissue which is an observation target is condensed by the optical system and introduced into the image pickup element. In the image pickup unit 10112, the incident observation light is photoelectrically converted by the image pickup element, by which an image signal corresponding to the observation light is generated. The image signal generated by the image pickup unit 10112 is provided to the image processing unit 10113.

The image processing unit 10113 includes a processor such as a central processing unit (CPU) or a graphics processing unit (GPU) and performs various signal processes for an image signal generated by the image pickup unit 10112. The image processing unit 10113 provides the image signal for which the signal processes have been performed thereby as RAW data to the wireless communication unit 10114.

The wireless communication unit 10114 performs a predetermined process such as a modulation process for the image signal for which the signal processes have been performed by the image processing unit 10113 and transmits the resulting image signal to the external controlling apparatus 10200 through an antenna 10114A. Further, the wireless communication unit 10114 receives a control signal relating to driving control of the capsule type endoscope 10100 from the external controlling apparatus 10200 through the antenna 10114A. The wireless communication unit 10114 provides the control signal received from the external controlling apparatus 10200 to the control unit 10117.

The power feeding unit 10115 includes an antenna coil for power reception, a power regeneration circuit for regenerating electric power from current generated in the antenna coil, a voltage booster circuit and so forth. The power feeding unit 10115 generates electric power using the principle of non-contact charging.

The power supply unit 10116 includes a secondary battery and stores electric power generated by the power feeding unit 10115. In FIG. 15, in order to avoid complicated illustration, an arrow mark indicative of a supply destination of electric power from the power supply unit 10116 and so forth are omitted. However, electric power stored in the power supply unit 10116 is supplied to and can be used to drive the light source unit 10111, the image pickup unit 10112, the image processing unit 10113, the wireless communication unit 10114 and the control unit 10117.

The control unit 10117 includes a processor such as a CPU and suitably controls driving of the light source unit 10111, the image pickup unit 10112, the image processing unit 10113, the wireless communication unit 10114 and the power feeding unit 10115 in accordance with a control signal transmitted thereto from the external controlling apparatus 10200.

The external controlling apparatus 10200 includes a processor such as a CPU or a GPU, a microcomputer, a control board or the like in which a processor and a storage element such as a memory are mixedly incorporated. The external controlling apparatus 10200 transmits a control signal to the control unit 10117 of the capsule type endoscope 10100 through an antenna 10200A to control operation of the capsule type endoscope 10100. In the capsule type endoscope 10100, an irradiation condition of light upon an observation target of the light source unit 10111 can be changed, for example, in accordance with a control signal from the external controlling apparatus 10200. Further, an image pickup condition (for example, a frame rate, an exposure value or the like of the image pickup unit 10112) can be changed in accordance with a control signal from the external controlling apparatus 10200. Further, the substance of processing by the image processing unit 10113 or a condition for transmitting an image signal from the wireless communication unit 10114 (for example, a transmission interval, a transmission image number or the like) may be changed in accordance with a control signal from the external controlling apparatus 10200.

Further, the external controlling apparatus 10200 performs various image processes for an image signal transmitted thereto from the capsule type endoscope 10100 to generate image data for displaying a picked up in-vivo image on the display apparatus. As the image processes, various signal processes can be performed such as, for example, a development process (demosaic process), an image quality improving process (bandwidth enhancement process, a super-resolution process, a noise reduction (NR) process and/or image stabilization process) and/or an enlargement process (electronic zooming process). The external controlling apparatus 10200 controls driving of the display apparatus to cause the display apparatus to display a picked up in-vivo image on the basis of generated image data. Alternatively, the external controlling apparatus 10200 may also control a recording apparatus (not depicted) to record generated image data or control a printing apparatus (not depicted) to output generated image data by printing.

The above has described the example of the in-vivo information acquisition system to which the technology according to the present disclosure may be applied. The technology according to the present disclosure may be applied, for example, to the image pickup unit 10112 among the components described above. This increases the detection accuracy.

### <Practical Application Example to Endoscopic Surgery System>

The technology (the present technology) according to the present disclosure is applicable to a variety of products. For example, the technology according to the present disclosure may be applied to an endoscopic surgery system.

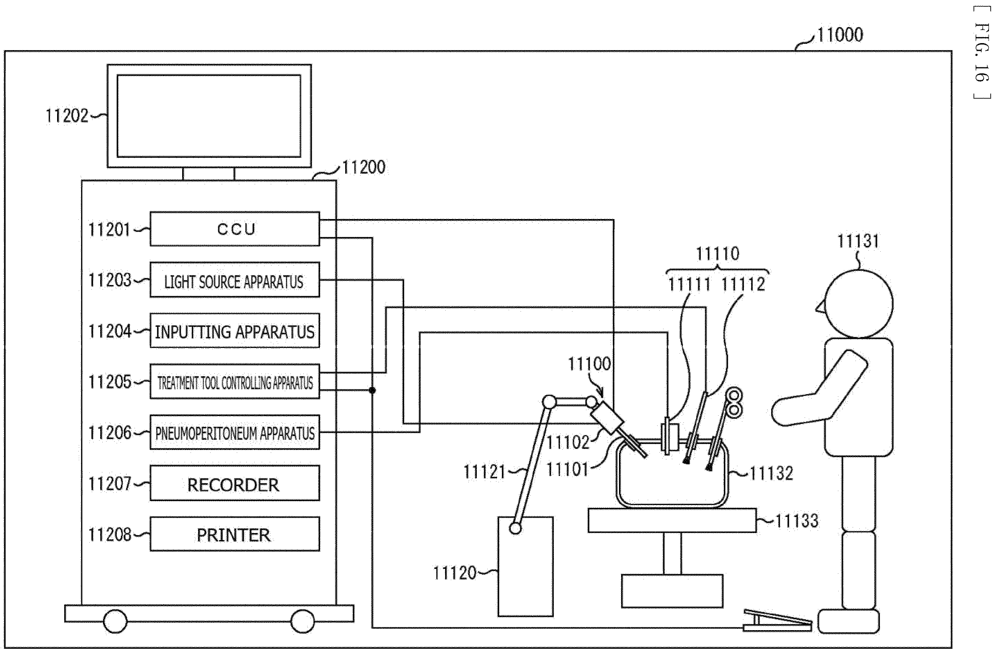

FIG. 16 is a view depicting an example of a schematic configuration of an endoscopic surgery system to which the technology according to an embodiment of the present disclosure (present technology) can be applied.

In FIG. 16, a state is illustrated in which a surgeon (medical doctor) 11131 is using an endoscopic surgery system 11000 to perform surgery for a patient 11132 on a patient bed 11133. As depicted, the endoscopic surgery system 11000 includes an endoscope 11100, other surgical tools 11110 such as a pneumoperitoneum tube 11111 and an energy device 11112, a supporting arm apparatus 11120 which supports the endoscope 11100 thereon, and a cart 11200 on which various apparatus for endoscopic surgery are mounted.

The endoscope 11100 includes a lens barrel 11101 having a region of a predetermined length from a distal end thereof to be inserted into a body cavity of the patient 11132, and a camera head 11102 connected to a proximal end of the lens barrel 11101. In the example depicted, the endoscope 11100 is depicted which includes as a rigid endoscope having the lens barrel 11101 of the hard type. However, the endoscope 11100 may otherwise be included as a flexible endoscope having the lens barrel 11101 of the flexible type.

The lens barrel 11101 has, at a distal end thereof, an opening in which an objective lens is fitted. A light source apparatus 11203 is connected to the endoscope 11100 such that light generated by the light source apparatus 11203 is introduced to a distal end of the lens barrel 11101 by a light guide extending in the inside of the lens barrel 11101 and is irradiated toward an observation target in a body cavity of the patient 11132 through the objective lens. It is to be noted that the endoscope 11100 may be a forward-viewing endoscope or may be an oblique-viewing endoscope or a side-viewing endoscope.

An optical system and an image pickup element are provided in the inside of the camera head 11102 such that reflected light (observation light) from the observation target is condensed on the image pickup element by the optical system. The observation light is photo-electrically converted by the image pickup element to generate an electric signal corresponding to the observation light, namely, an image signal corresponding to an observation image. The image signal is transmitted as RAW data to a CCU 11201.

The CCU 11201 includes a central processing unit (CPU), a graphics processing unit (GPU) or the like and integrally controls operation of the endoscope 11100 and a display apparatus 11202. Further, the CCU 11201 receives an image signal from the camera head 11102 and performs, for the image signal, various image processes for displaying an image based on the image signal such as, for example, a development process (demosaic process).

The display apparatus 11202 displays thereon an image based on an image signal, for which the image processes have been performed by the CCU 11201, under the control of the CCU 11201.

The light source apparatus 11203 includes a light source such as, for example, a light emitting diode (LED) and supplies irradiation light upon imaging of a surgical region to the endoscope 11100.

An inputting apparatus 11204 is an input interface for the endoscopic surgery system 11000. A user can perform inputting of various kinds of information or instruction inputting to the endoscopic surgery system 11000 through the inputting apparatus 11204. For example, the user would input an instruction or a like to change an image pickup condition (type of irradiation light, magnification, focal distance or the like) by the endoscope 11100.

A treatment tool controlling apparatus 11205 controls driving of the energy device 11112 for cautery or incision of a tissue, sealing of a blood vessel or the like. A pneumoperitoneum apparatus 11206 feeds gas into a body cavity of the patient 11132 through the pneumoperitoneum tube 11111 to inflate the body cavity in order to secure the field of view of the endoscope 11100 and secure the working space for the surgeon. A recorder 11207 is an apparatus capable of recording various kinds of information relating to surgery. A printer 11208 is an apparatus capable of printing various kinds of information relating to surgery in various forms such as a text, an image or a graph.

It is to be noted that the light source apparatus 11203 which supplies irradiation light when a surgical region is to be imaged to the endoscope 11100 may include a white light source which includes, for example, an LED, a laser light source or a combination of them. Where a white light source includes a combination of red, green, and blue (RGB) laser light sources, since the output intensity and the output timing can be controlled with a high degree of accuracy for each color (each wavelength), adjustment of the white balance of a picked up image can be performed by the light source apparatus 11203. Further, in this case, if laser beams from the respective RGB laser light sources are irradiated time-divisionally on an observation target and driving of the image pickup elements of the camera head 11102 are controlled in synchronism with the irradiation timings. Then images individually corresponding to the R, G and B colors can be also picked up time-divisionally. According to this method, a color image can be obtained even if color filters are not provided for the image pickup element.

Further, the light source apparatus 11203 may be controlled such that the intensity of light to be outputted is changed for each predetermined time. By controlling driving of the image pickup element of the camera head 11102 in synchronism with the timing of the change of the intensity of light to acquire images time-divisionally and synthesizing the images, an image of a high dynamic range free from underexposed blocked up shadows and overexposed highlights can be created.

Further, the light source apparatus 11203 may be configured to supply light of a predetermined wavelength band ready for special light observation. In special light observation, for example, by utilizing the wavelength dependency of absorption of light in a body tissue to irradiate light of a narrow band in comparison with irradiation light upon ordinary observation (namely, white light), narrow band observation (narrow band imaging) of imaging a predetermined tissue such as a blood vessel of a superficial portion of the mucous membrane or the like in a high contrast is performed. Alternatively, in special light observation, fluorescent observation for obtaining an image from fluorescent light generated by irradiation of excitation light may be performed. In fluorescent observation, it is possible to perform observation of fluorescent light from a body tissue by irradiating excitation light on the body tissue (autofluorescence observation) or to obtain a fluorescent light image by locally injecting a reagent such as indocyanine green (ICG) into a body tissue and irradiating excitation light corresponding to a fluorescent light wavelength of the reagent upon the body tissue. The light source apparatus 11203 can be configured to supply such narrow-band light and/or excitation light suitable for special light observation as described above.

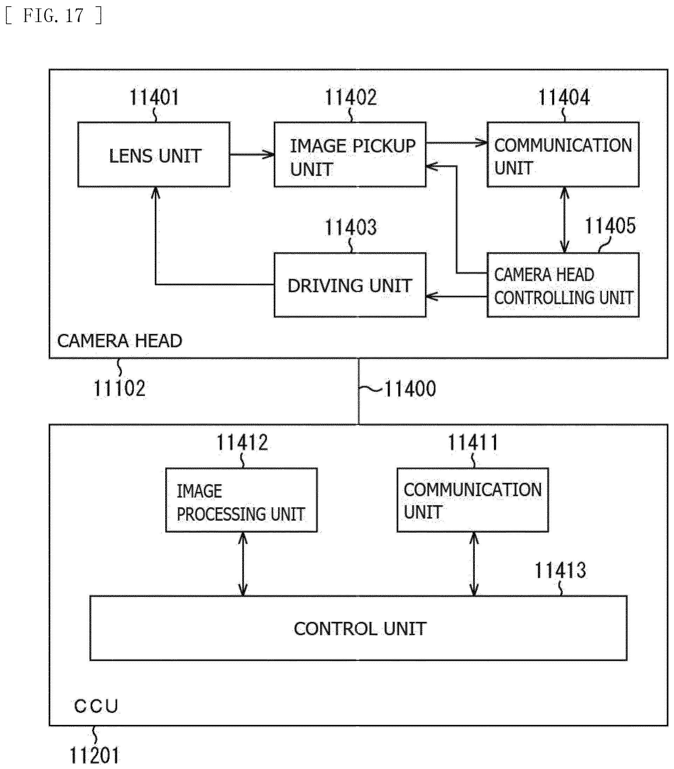

FIG. 17 is a block diagram depicting an example of a functional configuration of the camera head 11102 and the CCU 11201 depicted in FIG. 16.

The camera head 11102 includes a lens unit 11401, an image pickup unit 11402, a driving unit 11403, a communication unit 11404 and a camera head controlling unit 11405. The CCU 11201 includes a communication unit 11411, an image processing unit 11412 and a control unit 11413. The camera head 11102 and the CCU 11201 are connected for communication to each other by a transmission cable 11400.

The lens unit 11401 is an optical system, provided at a connecting location to the lens barrel 11101. Observation light taken in from a distal end of the lens barrel 11101 is guided to the camera head 11102 and introduced into the lens unit 11401. The lens unit 11401 includes a combination of a plurality of lenses including a zoom lens and a focusing lens.

The number of image pickup elements which is included by the image pickup unit 11402 may be one (single-plate type) or a plural number (multi-plate type). Where the image pickup unit 11402 is configured as that of the multi-plate type, for example, image signals corresponding to respective R, G and B are generated by the image pickup elements, and the image signals may be synthesized to obtain a color image. The image pickup unit 11402 may also be configured so as to have a pair of image pickup elements for acquiring respective image signals for the right eye and the left eye ready for three dimensional (3D) display. If 3D display is performed, then the depth of a living body tissue in a surgical region can be comprehended more accurately by the surgeon 11131. It is to be noted that, where the image pickup unit 11402 is configured as that of stereoscopic type, a plurality of systems of lens units 11401 are provided corresponding to the individual image pickup elements.

Further, the image pickup unit 11402 may not necessarily be provided on the camera head 11102. For example, the image pickup unit 11402 may be provided immediately behind the objective lens in the inside of the lens barrel 11101.

The driving unit 11403 includes an actuator and moves the zoom lens and the focusing lens of the lens unit 11401 by a predetermined distance along an optical axis under the control of the camera head controlling unit 11405. Consequently, the magnification and the focal point of a picked up image by the image pickup unit 11402 can be adjusted suitably.

The communication unit 11404 includes a communication apparatus for transmitting and receiving various kinds of information to and from the CCU 11201. The communication unit 11404 transmits an image signal acquired from the image pickup unit 11402 as RAW data to the CCU 11201 through the transmission cable 11400.

In addition, the communication unit 11404 receives a control signal for controlling driving of the camera head 11102 from the CCU 11201 and supplies the control signal to the camera head controlling unit 11405. The control signal includes information relating to image pickup conditions such as, for example, information that a frame rate of a picked up image is designated, information that an exposure value upon image picking up is designated and/or information that a magnification and a focal point of a picked up image are designated.

It is to be noted that the image pickup conditions such as the frame rate, exposure value, magnification or focal point may be designated by the user or may be set automatically by the control unit 11413 of the CCU 11201 on the basis of an acquired image signal. In the latter case, an auto exposure (AE) function, an auto focus (AF) function and an auto white balance (AWB) function are incorporated in the endoscope 11100.

The camera head controlling unit 11405 controls driving of the camera head 11102 on the basis of a control signal from the CCU 11201 received through the communication unit 11404.

The communication unit 11411 includes a communication apparatus for transmitting and receiving various kinds of information to and from the camera head 11102. The communication unit 11411 receives an image signal transmitted thereto from the camera head 11102 through the transmission cable 11400.

Further, the communication unit 11411 transmits a control signal for controlling driving of the camera head 11102 to the camera head 11102. The image signal and the control signal can be transmitted by electrical communication, optical communication or the like.

The image processing unit 11412 performs various image processes for an image signal in the form of RAW data transmitted thereto from the camera head 11102.

The control unit 11413 performs various kinds of control relating to image picking up of a surgical region or the like by the endoscope 11100 and display of a picked up image obtained by image picking up of the surgical region or the like. For example, the control unit 11413 creates a control signal for controlling driving of the camera head 11102.

Further, the control unit 11413 controls, on the basis of an image signal for which image processes have been performed by the image processing unit 11412, the display apparatus 11202 to display a picked up image in which the surgical region or the like is imaged. Thereupon, the control unit 11413 may recognize various objects in the picked up image using various image recognition technologies. For example, the control unit 11413 can recognize a surgical tool such as forceps, a particular living body region, bleeding, mist when the energy device 11112 is used and so forth by detecting the shape, color and so forth of edges of objects included in a picked up image. The control unit 11413 may cause, when it controls the display apparatus 11202 to display a picked up image, various kinds of surgery supporting information to be displayed in an overlapping manner with an image of the surgical region using a result of the recognition. Where surgery supporting information is displayed in an overlapping manner and presented to the surgeon 11131, the burden on the surgeon 11131 can be reduced and the surgeon 11131 can proceed with the surgery with certainty.

The transmission cable 11400 which connects the camera head 11102 and the CCU 11201 to each other is an electric signal cable ready for communication of an electric signal, an optical fiber ready for optical communication or a composite cable ready for both of electrical and optical communications.

Here, while, in the example depicted, communication is performed by wired communication using the transmission cable 11400, the communication between the camera head 11102 and the CCU 11201 may be performed by wireless communication.

The above has described the example of the endoscopic surgery system to which the technology according to the present disclosure may be applied. The technology according to the present disclosure may be applied to the image pickup unit 11402 among the components described above. The application of the technology according to the present disclosure to the image pickup unit 11402 increases the detection accuracy.

It is to be noted that the endoscopic surgery system has been described here as an example, but the technology according to the present disclosure may be additionally applied, for example, to a microscopic surgery system or the like.

### <Practical Application Example to Mobile Body>

The technology according to the present disclosure is applicable to a variety of products. For example, the technology according to the present disclosure may be achieved as a device mounted on any type of mobile body such as a vehicle, an electric vehicle, a hybrid electric vehicle, a motorcycle, a bicycle, a personal mobility, an airplane, a drone, a vessel, a robot, a construction machine, or an agricultural machine (tractor).

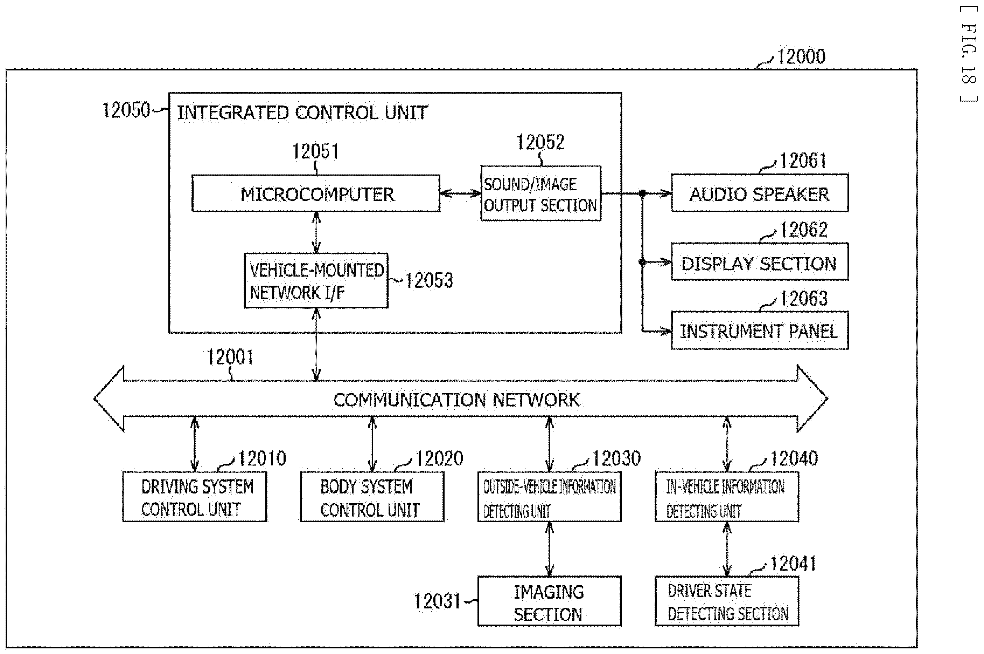

FIG. 18 is a block diagram depicting an example of schematic configuration of a vehicle control system as an example of a mobile body control system to which the technology according to an embodiment of the present disclosure can be applied.

The vehicle control system 12000 includes a plurality of electronic control units connected to each other via a communication network 12001. In the example depicted in FIG. 18, the vehicle control system 12000 includes a driving system control unit 12010, a body system control unit 12020, an outside-vehicle information detecting unit 12030, an in-vehicle information detecting unit 12040, and an integrated control unit 12050. In addition, a microcomputer 12051, a sound/image output section 12052, and a vehicle-mounted network interface (I/F) 12053 are illustrated as a functional configuration of the integrated control unit 12050.

The driving system control unit 12010 controls the operation of devices related to the driving system of the vehicle in accordance with various kinds of programs. For example, the driving system control unit 12010 functions as a control device for a driving force generating device for generating the driving force of the vehicle, such as an internal combustion engine, a driving motor, or the like, a driving force transmitting mechanism for transmitting the driving force to wheels, a steering mechanism for adjusting the steering angle of the vehicle, a braking device for generating the braking force of the vehicle, and the like.

The body system control unit 12020 controls the operation of various kinds of devices provided to a vehicle body in accordance with various kinds of programs. For example, the body system control unit 12020 functions as a control device for a keyless entry system, a smart key system, a power window device, or various kinds of lamps such as a headlamp, a backup lamp, a brake lamp, a turn signal, a fog lamp, or the like. In this case, radio waves transmitted from a mobile device as an alternative to a key or signals of various kinds of switches can be input to the body system control unit 12020. The body system control unit 12020 receives these input radio waves or signals, and controls a door lock device, the power window device, the lamps, or the like of the vehicle.

The outside-vehicle information detecting unit 12030 detects information about the outside of the vehicle including the vehicle control system 12000. For example, the outside-vehicle information detecting unit 12030 is connected with an imaging section 12031. The outside-vehicle information detecting unit 12030 makes the imaging section 12031 image an image of the outside of the vehicle, and receives the imaged image. On the basis of the received image, the outside-vehicle information detecting unit 12030 may perform processing of detecting an object such as a human, a vehicle, an obstacle, a sign, a character on a road surface, or the like, or processing of detecting a distance thereto.

The imaging section 12031 is an optical sensor that receives light, and which outputs an electric signal corresponding to a received light amount of the light. The imaging section 12031 can output the electric signal as an image, or can output the electric signal as information about a measured distance. In addition, the light received by the imaging section 12031 may be visible light, or may be invisible light such as infrared rays or the like.

The in-vehicle information detecting unit 12040 detects information about the inside of the vehicle. The in-vehicle information detecting unit 12040 is, for example, connected with a driver state detecting section 12041 that detects the state of a driver. The driver state detecting section 12041, for example, includes a camera that images the driver. On the basis of detection information input from the driver state detecting section 12041, the in-vehicle information detecting unit 12040 may calculate a degree of fatigue of the driver or a degree of concentration of the driver, or may determine whether the driver is dozing.

The microcomputer 12051 can calculate a control target value for the driving force generating device, the steering mechanism, or the braking device on the basis of the information about the inside or outside of the vehicle which information is obtained by the outside-vehicle information detecting unit 12030 or the in-vehicle information detecting unit 12040, and output a control command to the driving system control unit 12010. For example, the microcomputer 12051 can perform cooperative control intended to implement functions of an advanced driver assistance system (ADAS) which functions include collision avoidance or shock mitigation for the vehicle, following driving based on a following distance, vehicle speed maintaining driving, a warning of collision of the vehicle, a warning of deviation of the vehicle from a lane, or the like.

In addition, the microcomputer 12051 can perform cooperative control intended for automatic driving, which makes the vehicle to travel autonomously without depending on the operation of the driver, or the like, by controlling the driving force generating device, the steering mechanism, the braking device, or the like on the basis of the information about the outside or inside of the vehicle which information is obtained by the outside-vehicle information detecting unit 12030 or the in-vehicle information detecting unit 12040.

In addition, the microcomputer 12051 can output a control command to the body system control unit 12020 on the basis of the information about the outside of the vehicle which information is obtained by the outside-vehicle information detecting unit 12030. For example, the microcomputer 12051 can perform cooperative control intended to prevent a glare by controlling the headlamp so as to change from a high beam to a low beam, for example, in accordance with the position of a preceding vehicle or an oncoming vehicle detected by the outside-vehicle information detecting unit 12030.

The sound/image output section 12052 transmits an output signal of at least one of a sound and an image to an output device capable of visually or auditorily notifying information to an occupant of the vehicle or the outside of the vehicle. In the example of FIG. 18, an audio speaker 12061, a display section 12062, and an instrument panel 12063 are illustrated as the output device. The display section 12062 may, for example, include at least one of an on-board display and a head-up display.

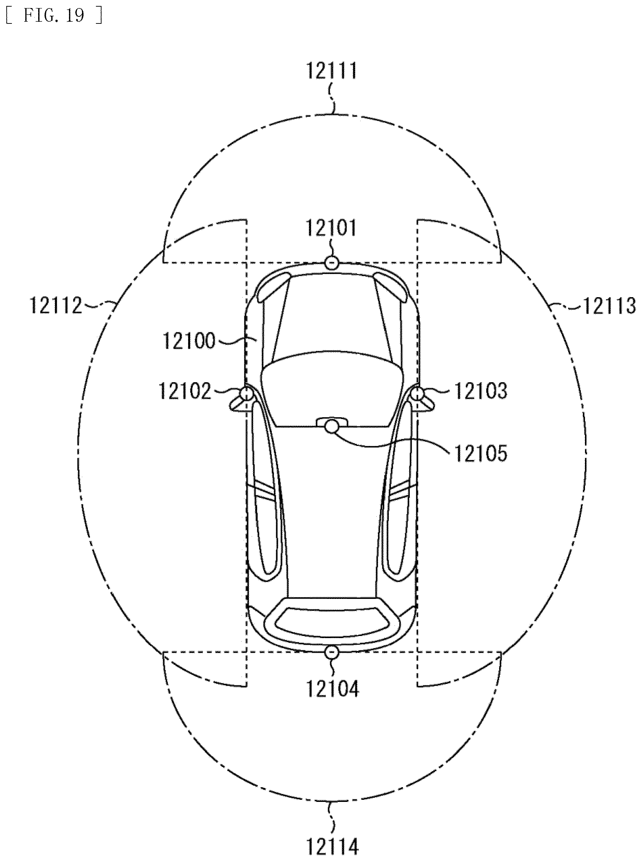

FIG. 19 is a diagram depicting an example of the installation position of the imaging section 12031.

In FIG. 19, the imaging section 12031 includes imaging sections 12101, 12102, 12103, 12104, and 12105.

The imaging sections 12101, 12102, 12103, 12104, and 12105 are, for example, disposed at positions on a front nose, sideview mirrors, a rear bumper, and a back door of the vehicle 12100 as well as a position on an upper portion of a windshield within the interior of the vehicle. The imaging section 12101 provided to the front nose and the imaging section 12105 provided to the upper portion of the windshield within the interior of the vehicle obtain mainly an image of the front of the vehicle 12100. The imaging sections 12102 and 12103 provided to the sideview mirrors obtain mainly an image of the sides of the vehicle 12100. The imaging section 12104 provided to the rear bumper or the back door obtains mainly an image of the rear of the vehicle 12100. The imaging section 12105 provided to the upper portion of the windshield within the interior of the vehicle is used mainly to detect a preceding vehicle, a pedestrian, an obstacle, a signal, a traffic sign, a lane, or the like.

Incidentally, FIG. 19 depicts an example of photographing ranges of the imaging sections 12101 to 12104. An imaging range 12111 represents the imaging range of the imaging section 12101 provided to the front nose. Imaging ranges 12112 and 12113 respectively represent the imaging ranges of the imaging sections 12102 and 12103 provided to the sideview mirrors. An imaging range 12114 represents the imaging range of the imaging section 12104 provided to the rear bumper or the back door. A bird's-eye image of the vehicle 12100 as viewed from above is obtained by superimposing image data imaged by the imaging sections 12101 to 12104, for example.

At least one of the imaging sections 12101 to 12104 may have a function of obtaining distance information. For example, at least one of the imaging sections 12101 to 12104 may be a stereo camera constituted of a plurality of imaging elements, or may be an imaging element having pixels for phase difference detection.

For example, the microcomputer 12051 can determine a distance to each three-dimensional object within the imaging ranges 12111 to 12114 and a temporal change in the distance (relative speed with respect to the vehicle 12100) on the basis of the distance information obtained from the imaging sections 12101 to 12104, and thereby extract, as a preceding vehicle, a nearest three-dimensional object in particular that is present on a traveling path of the vehicle 12100 and which travels in substantially the same direction as the vehicle 12100 at a predetermined speed (for example, equal to or more than 0 km/hour). Further, the microcomputer 12051 can set a following distance to be maintained in front of a preceding vehicle in advance, and perform automatic brake control (including following stop control), automatic acceleration control (including following start control), or the like. It is thus possible to perform cooperative control intended for automatic driving that makes the vehicle travel autonomously without depending on the operation of the driver or the like.

For example, the microcomputer 12051 can classify three-dimensional object data on three-dimensional objects into three-dimensional object data of a two-wheeled vehicle, a standard-sized vehicle, a large-sized vehicle, a pedestrian, a utility pole, and other three-dimensional objects on the basis of the distance information obtained from the imaging sections 12101 to 12104, extract the classified three-dimensional object data, and use the extracted three-dimensional object data for automatic avoidance of an obstacle. For example, the microcomputer 12051 identifies obstacles around the vehicle 12100 as obstacles that the driver of the vehicle 12100 can recognize visually and obstacles that are difficult for the driver of the vehicle 12100 to recognize visually. Then, the microcomputer 12051 determines a collision risk indicating a risk of collision with each obstacle. In a situation in which the collision risk is equal to or higher than a set value and there is thus a possibility of collision, the microcomputer 12051 outputs a warning to the driver via the audio speaker 12061 or the display section 12062, and performs forced deceleration or avoidance steering via the driving system control unit 12010. The microcomputer 12051 can thereby assist in driving to avoid collision.

At least one of the imaging sections 12101 to 12104 may be an infrared camera that detects infrared rays. The microcomputer 12051 can, for example, recognize a pedestrian by determining whether or not there is a pedestrian in imaged images of the imaging sections 12101 to 12104. Such recognition of a pedestrian is, for example, performed by a procedure of extracting characteristic points in the imaged images of the imaging sections 12101 to 12104 as infrared cameras and a procedure of determining whether or not it is the pedestrian by performing pattern matching processing on a series of characteristic points representing the contour of the object. When the microcomputer 12051 determines that there is a pedestrian in the imaged images of the imaging sections 12101 to 12104, and thus recognizes the pedestrian, the sound/image output section 12052 controls the display section 12062 so that a square contour line for emphasis is displayed so as to be superimposed on the recognized pedestrian. The sound/image output section 12052 may also control the display section 12062 so that an icon or the like representing the pedestrian is displayed at a desired position.

The description has been given with reference to the first to fourth embodiments, the modification examples 1 to 3, the application examples, and the practical application examples, but the contents of the present disclosure are not limited to the embodiments or the like described above. It is possible to make a variety of modifications.

It is to be noted that the effects described herein are merely examples, but not limitative. In addition, there may be other effects.

It is to be noted that the present disclosure may have the following configurations. The present technology having the following configurations provides the light transmissive substrate on the light receiving surface of the sensor substrate. This makes it possible to use this light transmissive substrate as a lens. The light transmissive substrate has the flat region and the uneven region on the other surface opposite to the one surface opposed to the light receiving surface. The flat region has the flat front surface. The concave section or the convex section is formed in the uneven region. This makes it possible, for example, in the camera module to simplify the design of the lens module provided on the light receiving surface side of the sensor module. It is thus possible to achieve a reduction in the height of the camera module.

## Claims

1. A sensor module (2) comprising:
a sensor substrate (10, 70) having a light receiving surface (11S1) on which a plurality of light receiving elements (12) is arranged; and
a light transmissive substrate (20) that is provided on the light receiving surface (11S1), the light transmissive substrate (20) having a flat region (20B) and an uneven region (20A) on another surface (20S1) opposite to one surface (20S2) opposed to the light receiving surface (11S1) of the sensor substrate (10), the flat region (20B) having a flat front surface, the uneven region (20A) having a concave section (20X1) or a convex section (20X2) formed therein, wherein
the light transmissive substrate (20) has a thickness that serves as a distance (h) between the opposed surface (20S2) to the sensor substrate (10, 70) and the flat region (20B) on the another surface (20S1), **characterised in that**
the thickness is equal to or greater than a thickness of the sensor substrate (10, 70), and
the light transmissive substrate (20) includes a flattening member (21) on the other surface (20S1), the flattening member (21) filling the concave section (20X1) or the convex section (20X2) formed in the uneven region (20A).

2. The sensor module (1, 2, 3, 4, 5, 6, 7) according to claim 1, wherein the light transmissive substrate (20) has the uneven region (20A) in a middle and has the flat region (20A) around the uneven region (20B).

3. The sensor module (1, 2, 3, 4, 5, 6, 7) according to any one of the preceding claims, wherein
the sensor substrate (10) has a light receiving region (10A) in which the plurality of light receiving elements (12) is arranged,
the uneven region (20A) is provided at a position opposed to the light receiving region (10A), and
the light receiving region (10A) is covered with the uneven region (20A) in a plan view.

4. The sensor module (2) according to any one of the preceding claims, wherein the flattening member (21) has a refractive index different from a refractive index of the light transmissive substrate (20).

5. The sensor module (1, 2, 3, 4, 7) according to any one of the preceding claims, wherein the sensor substrate (10) and the light transmissive substrate (20) are bonded together with a resin layer (30, 40) interposed in between.

6. The sensor module (4) according to claim 5, wherein
the sensor substrate (10) has a light receiving region (10A) in which the plurality of light receiving elements (12) is arranged, and
the resin layer (40) has a hollow structure (40X) at a position opposed to the light receiving region (10A).

7. The sensor module (1) according to claim 1, wherein the light transmissive substrate (20) includes a light shielding film (23) on a side surface or on the flat region (20B).

8. The sensor module (3) according to claim 1, wherein the light transmissive substrate (20) has a functional layer (22) formed on the other surface (20S1), and the functional layer (22) includes at least one of an anti-reflection film, an infrared cut filter, or a bandpass filter.

9. The sensor module (1, 2, 3, 4, 5, 6, 7) according to any one of the preceding claims, wherein the light transmissive substrate (20) includes any of a glass substrate and an acrylic resin.

10. The sensor module (1, 2, 3, 4, 5, 6, 7) according to any one of the preceding claims, wherein the sensor substrate (10) includes a wiring layer on a surface opposite to the light receiving surface (11S1).

11. The sensor module (1, 2, 3, 4, 5, 6, 7) according to any one of the preceding claims, wherein the light transmissive substrate (20) is larger than the sensor substrate (10, 70).

12. The sensor module (5) according to any one of the preceding claims, further comprising a wiring substrate on which a wiring line (51) is formed, wherein
the wiring substrate is disposed on a periphery of the sensor substrate (10) in a plan view between the sensor substrate (10) and the light transmissive substrate (20).

13. An electronic apparatus (100) comprising the sensor module (1, 2, 3, 4, 5) according to any one of the preceding claims.

## Patentansprüche

1. Sensormodul (2), umfassend:
ein Sensorsubstrat (10, 70), das eine Lichtempfangsoberfläche (11S1) aufweist, auf der eine Vielzahl von Lichtempfangselementen (12) angeordnet ist; und
ein lichtdurchlässiges Substrat (20), das auf der Lichtempfangsoberfläche (11S1) bereitgestellt ist, wobei das lichtdurchlässige Substrat (20) einen flachen Bereich (20B) und einen unebenen Bereich (20A) auf einer anderen Oberfläche (20S1) gegenüber einer Oberfläche (20S2) aufweist, die der Lichtempfangsoberfläche (11S1) des Sensorsubstrats (10) gegenüberliegt, wobei der flache Bereich (20B) eine flache Vorderoberfläche aufweist und der unebene Bereich (20A) einen darin gebildeten konkaven Abschnitt (20X1) oder einen konvexen Abschnitt (20X2) aufweist, wobei
das lichtdurchlässige Substrat (20) eine Dicke aufweist, die als Abstand (h) zwischen der dem Sensorsubstrat (10, 70) gegenüberliegenden Oberfläche (20S2) und dem flachen Bereich (20B) auf der anderen Oberfläche (20S1) dient, **dadurch gekennzeichnet, dass** die Dicke gleich oder größer als eine Dicke des Sensorsubstrats (10, 70) ist, und
das lichtdurchlässige Substrat (20) ein Abflachungselement (21) auf der anderen Oberfläche (20S1) einschließt, wobei das Abflachungselement (21) den konkaven Abschnitt (20X1) oder den konvexen Abschnitt (20X2), der in dem unebenen Bereich (20A) gebildet ist, ausfüllt.

2. Sensormodul (1, 2, 3, 4, 5, 6, 7) nach Anspruch 1, wobei das lichtdurchlässige Substrat (20) in der Mitte den unebenen Bereich (20A) aufweist und um den unebenen Bereich (20B) herum den flachen Bereich (20A) aufweist.

3. Sensormodul (1, 2, 3, 4, 5, 6, 7) nach einem der vorstehenden Ansprüche, wobei
das Sensorsubstrat (10) einen Lichtempfangsbereich (10A) aufweist, in dem die Vielzahl von Lichtempfangselementen (12) angeordnet ist,
der unebene Bereich (20A) an einer Position gegenüber dem Lichtempfangsbereich (10A) bereitgestellt ist, und
der Lichtempfangsbereich (10A) in einer Draufsicht mit dem unebenen Bereich (20A) bedeckt ist.

4. Sensormodul (2) nach einem der vorstehenden Ansprüche, wobei das Abflachungselement (21) einen Brechungsindex aufweist, der sich von einem Brechungsindex des lichtdurchlässigen Substrats (20) unterscheidet.

5. Sensormodul (1, 2, 3, 4, 7) nach einem der vorstehenden Ansprüche, wobei das Sensorsubstrat (10) und das lichtdurchlässige Substrat (20) mit einer dazwischen angeordneten Harzschicht (30, 40) miteinander verbunden sind.

6. Sensormodul (4) nach Anspruch 5, wobei
das Sensorsubstrat (10) einen Lichtempfangsbereich (10A) aufweist, in dem die Vielzahl von Lichtempfangselementen (12) angeordnet ist, und
die Harzschicht (40) an einer dem Lichtempfangsbereich (10A) gegenüberliegenden Position eine Hohlstruktur (40X) aufweist.

7. Sensormodul (1) nach Anspruch 1, wobei das lichtdurchlässige Substrat (20) einen Lichtabschirmfilm (23) auf einer Seitenoberfläche oder auf dem flachen Bereich (20B) einschließt.

8. Sensormodul (3) nach Anspruch 1, wobei das lichtdurchlässige Substrat (20) eine Funktionsschicht (22) aufweist, die auf der anderen Oberfläche (20S1) gebildet ist, und die Funktionsschicht (22) mindestens eines von einem Antireflexionsfilm, einem Infrarot-Sperrfilter oder einem Bandpassfilter einschließt.

9. Sensormodul (1, 2, 3, 4, 5, 6, 7) nach einem der vorstehenden Ansprüche, wobei das lichtdurchlässige Substrat (20) ein beliebiges von einem Glassubstrat und einem Acrylharz einschließt.

10. Sensormodul (1, 2, 3, 4, 5, 6, 7) nach einem der vorstehenden Ansprüche, wobei das Sensorsubstrat (10) eine Verdrahtungsschicht auf einer der Lichtempfangsoberfläche (11S1) gegenüberliegenden Oberfläche einschließt.

11. Sensormodul (1, 2, 3, 4, 5, 6, 7) nach einem der vorstehenden Ansprüche, wobei das lichtdurchlässige Substrat (20) größer ist als das Sensorsubstrat (10, 70).

12. Sensormodul (5) nach einem der vorstehenden Ansprüche, ferner umfassend ein Verdrahtungssubstrat, auf dem eine Verdrahtungsleitung (51) gebildet ist, wobei
das Verdrahtungssubstrat in einer Draufsicht an einem Umfang des Sensorsubstrats (10) zwischen dem Sensorsubstrat (10) und dem lichtdurchlässigen Substrat (20) angeordnet ist.

13. Elektronische Einrichtung (100), umfassend das Sensormodul (1, 2, 3, 4, 5) nach einem der vorstehenden Ansprüche.

## Revendications

1. Module de capteur (2) comprenant :
un substrat de capteur (10, 70) ayant une surface de réception de lumière (11S1) sur laquelle sont agencés une pluralité d'éléments de réception de lumière (12) ; et
un substrat transmettant la lumière (20) qui est fourni sur la surface de réception de lumière (11S1), le substrat transmettant la lumière (20) ayant une région plate (20B) et une région irrégulière (20A) sur une autre surface (20S1) opposée à une surface (20S2) opposée à la surface de réception de lumière (11S1) du substrat de capteur (10), la région plate (20B) ayant une surface avant plate, la région irrégulière (20A) ayant une section concave (20X1) ou une section convexe (20X2) formée dans celle-ci, dans lequel
le substrat transmettant la lumière (20) a une épaisseur qui sert de distance (h) entre la surface opposée (20S2) au substrat de capteur (10, 70) et la région plate (20B) sur l'autre surface (20S1), **caractérisé en ce que** l'épaisseur est égale ou supérieure à l'épaisseur du substrat de capteur (10, 70), et
le substrat transmettant la lumière (20) comporte un organe d'aplatissement (21) sur l'autre surface (20S1), l'organe d'aplatissement (21) remplissant la section concave (20X1) ou la section convexe (20X2) formée dans la région irrégulière (20A).

2. Module de capteur (1, 2, 3, 4, 5, 6, 7) selon la revendication 1, dans lequel le substrat transmettant la lumière (20) a la région irrégulière (20A) dans un milieu et a la région plate (20A) autour de la région irrégulière (20B).

3. Module de capteur (1, 2, 3, 4, 5, 6, 7) selon l'une quelconque des revendications précédentes, dans lequel
le substrat de capteur (10) a une région de réception de lumière (10A) dans laquelle sont agencés la pluralité d'éléments de réception de lumière (12),
la région irrégulière (20A) est fournie au niveau d'une position opposée à la région de réception de lumière (10A), et
la région de réception de lumière (10A) est recouverte par la région irrégulière (20A) dans une vue en plan.

4. Module de capteur (2) selon l'une quelconque des revendications précédentes, dans lequel l'organe d'aplatissement (21) a un indice de réfraction différent d'un indice de réfraction du substrat transmettant la lumière (20).

5. Module de capteur (1, 2, 3, 4, 7) selon l'une quelconque des revendications précédentes, dans lequel le substrat de capteur (10) et le substrat transmettant la lumière (20) sont collés ensemble avec une couche de résine (30, 40) interposée entre les deux.

6. Module de capteur (4) selon la revendication 5, dans lequel
le substrat de capteur (10) a une région de réception de lumière (10A) dans laquelle sont agencés la pluralité d'éléments de réception de lumière (12), et
la couche de résine (40) a une structure creuse (40X) au niveau d'une position opposée à la région de réception de lumière (10A).

7. Module de capteur (1) selon la revendication 1, dans lequel le substrat transmettant la lumière (20) comporte un film de protection contre la lumière (23) sur une surface latérale ou sur la région plate (20B).

8. Module de capteur (3) selon la revendication 1, dans lequel le substrat transmettant la lumière (20) a une couche fonctionnelle (22) formée sur l'autre surface (20S1), et la couche fonctionnelle (22) comporte au moins l'un parmi un film antireflet, un filtre de coupure infrarouge, ou un filtre passe-bande.

9. Module de capteur (1, 2, 3, 4, 5, 6, 7) selon l'une quelconque des revendications précédentes, dans lequel le substrat transmettant la lumière (20) comporte un quelconque parmi un substrat en verre ou une résine acrylique.

10. Module de capteur (1, 2, 3, 4, 5, 6, 7) selon l'une quelconque des revendications précédentes, dans lequel le substrat de capteur (10) comporte une couche de câblage sur une surface opposée à la surface de réception de lumière (11S1).

11. Module de capteur (1, 2, 3, 4, 5, 6, 7) selon l'une quelconque des revendications précédentes, dans lequel le substrat transmettant la lumière (20) est plus grand que le substrat de capteur (10, 70).

12. Module de capteur (5) selon l'une quelconque des revendications précédentes, comprenant en outre un substrat de câblage sur lequel est formée une ligne de câblage (51), dans lequel
le substrat de câblage est disposé sur une périphérie du substrat de capteur (10) dans une vue en plan entre le substrat de capteur (10) et le substrat transmettant la lumière (20).

13. Appareil électronique (100) comprenant le module de capteur (1, 2, 3, 4, 5) selon l'une quelconque des revendications précédentes.
